# EUROPÄISCHE PATENTSCHRIFT

(11) **EP 0 883 602 B1**
(45) Veröffentlichungstag und Bekanntmachung des Hinweises auf die Patenterteilung: **04.09.2002**
(21) Anmeldenummer: 97904417.9
(22) Anmeldetag: 12.02.1997
(51) Int. Cl.: C07C 237/08, C07C 279/14, C07C 271/22, C12N 15/87, C12N 15/88, A61K 9/127

(54) **Lipidverbindungen**
Lipids
Composés lipidiques

(30) Priorität: 13.02.1996 DE 19605175
(43) Veröffentlichungstag der Anmeldung: 16.12.1998
(73) Patentinhaber: Sourovoi, Andrej, 103009 Moskau (RU)
(72) Erfinder: SOUROVOI, Andrej, 103009 Moscow (RU); JUNG, Guenther, D-72076 Tübingen (DE)
(74) Vertreter: Patentanwälte Ruff, Wilhelm, Beier, Dauster & Partner
(86) Internationale Anmeldenummer: EP9700629
(87) Internationale Veröffentlichungsnummer: WO97030024

(56) Entgegenhaltungen:
- WO-A-89/08098
- JP-A- 60 228 564
- JP-A- 61 037 892
- CHEMICAL ABSTRACTS, vol. 114, no. 9, 4.März 1991 Columbus, Ohio, US; abstract no. 76422, YAGI, KUNIO ET AL: "Introduction of DNA into mammalian cells with liposomes or lipid suspension" XP002032761 & JP 02 135 092 A (VITAMIN KENKYUSHO K. K., JAPAN) & RN:107066-27-3 Ethanaminium, 2-[bis[2-[(1-oxononadecyl)- oxy]ethyl]amino]-N,N,N-trimethyl-2-oxo-, bromide & RN:100993-84-8 Ethanaminium, 2-[bis[2-[(1-oxododecyl)oxy] ethyl]amino]-N,N,N-trimethyl-2-oxo-, chloride
- ACS SYMP. SER. (1986), 311(PHENOM. MIXED SURFACTANT SYST.), 270-82 CODEN: ACSMC8;ISSN: 0097-6156, 1986, XP000675553 SHIRAHAMA, KEISHIRO ET AL: "The growth of molecular assemblies in mild surfactant solutions"
- PATENT ABSTRACTS OF JAPAN vol. 95, no. 3, 28.April 1995 & JP 06 340598 A (LION CORP.)
- MEMOIRS OF THE FACULTY OF ENGINEERING, KYUSHU UNIVERSITY, Bd. 46, Nr. 2, 1986, Seiten 221-243, XP000675628 T. KUNITAKE ET AL.: "dsc studies of the phase transition behavior of synthetic bilayer membranes. Part I Bilayer membranes of double chain amphiphiles"

## Beschreibung

Die Erfindung betrifft Lipidverbindungen.

Liposome sind sphärische, in sich geschlossene Strukturen aus Lipiddoppelschichten, die in ihrem Inneren einen Teil des Lösemittels, in dem sie schwimmen, einlagern. Sie können aus einer oder mehreren konzentrischen Membranen bestehen und ihre Größe liegt im Bereich von einigen Nanometern bis einigen Dutzend Mikrometern.

Liposome sind hauptsächlich aus amphiphilen Molekülen gebildet, die durch eine hydrophile (oft polares Ende genannte) Gruppe und eine hydrophobe Gruppe (unpolares Ende) im selben Molekül gekennzeichnet sind. In den meisten Fällen sind liposombildende Moleküle nicht wasserlöslich. Unter bestimmten Umständen können sie jedoch kolloide Dispersionen bilden.

Liposome können groß oder klein sein und sie können aus einer bis mehreren hundert konzentrischen Doppelschichten ausgebildet sein. Bezüglich der Größe und Art der Schichten (Lamellen) können sie in mehrschichtige Vesikel (MLV, multi-lamellar vesicles), kleine einschichtige Vesikel (SUV, small uni-lamellar vesicles) und große einschichtige Vesikel (LUV, large uni-lamellar vesicles) eingeteilt werden.

SUV besitzen einen Durchmesser von 20 bis 600 nm und bestehen aus einer einzigen Lipiddoppelschicht, die die innere wäßrige Zone umgibt. LUV besitzen einen Durchmesser von 600 bis 30000 nm. MLV schwanken in ihrer Größe stark mit bis zu 10000 nm und enthalten mehr als eine Lipiddoppelschicht, daher sind sie in ihrer Struktur in mehrere Zonen geteilt.

Liposomen können auf zahlreichen Wegen hergestellt werden. Das Verfahren der sogenannten "Dünnschichthydratisierung" führt zur Bildung heterogener Dispersionen, die hauptsächlich MLV enthalten. Bei Verwendung geladener Lipidzusammensetzungen können ziemlich hohe Fraktionen von LUV hergestellt werden. Diese Dispersionen können weiter behandelt werden (mechanisch, elektrostatisch oder chemisch), um Lösungen von SUV herzustellen. Meist umfassen diese Verfahren eine Extrusion durch Filter mit Poren unterschiedlicher Durchmesser oder Schallbehandlung.

Alternativ können Liposome durch Lyophilisierung hergestellt werden, wobei der Lipidfilm dann in einem flüchtigen Lösemittel gelöst (zum Beispiel in tert-Butylalkohol), eingefroren und lyophilisiert wird.

In der Zeitschriften- und Patentliteratur wurden eine Reihe von Methoden zur Herstellung von Liposomen beschrieben wie: Szoka und Papahadjopoulos in: Ann. Rev. Biophys. Bioeng. 9 (1980), 467-508 sowie US-Patente Nr. 4229360, 4241046, 4235871.

Ein wichtiges Merkmal der Liposome ist ihre Fähigkeit, hydrophile oder hydrophobe Moleküle zu lösen, zu schützen und zu tragen. Für negativ geladene Arzneistoffe, einschließlich einiger Proteine, können positiv geladene Liposome verwendet werden. Es wurden Verbesserungen der Therapie beobachtet, trotz der bekannten Tatsache, daß positiv geladene Liposome toxisch wirken können.

In den letzten zwanzig Jahren wurden verschiedene DNA-Transfektionsverfahren entwickelt. Diese Verfahren umfassen Calciumphosphatfällung, das DEAE-Dextranverfahren, Elektroporation, Mikroinjektion, rezeptorvermittelte Endozytose, Liposome und Virusvektoren. Die meisten dieser Verfahren weisen jedoch einige gravierende Nachteile auf: sie sind entweder zu uneffizient, zu toxisch oder zu kompliziert und aufwendig für eine effektive Anwendung in biologischen und therapeutischen Behandlungsplänen in vitro und in vivo. Beispielsweise ist das am häufigsten in vitro angewendete Calciumphosphatfällungsverfahren zu uneffizient (durchschnittliche Transfektionsfrequenz 1 in 10⁴ Zellen). Elektroporation ist viel effizienter als das Calciumphosphatverfahren. Das Verfahren ist jedoch zu aggressiv (maximale Effizienz wird bei ungefähr 50 % Zelltötung erreicht) und außerdem erfordert dieses Verfahren eine Spezialapparatur. Mikroinjektion ist effizient, aber sie ist zu mühsam und nicht praktisch anzuwenden. Insgesamt können diese Verfahren nicht in vivo angewendet werden.

Ein Endozytoseverfahren mit Rezeptorvermittlung verwendet Polylysin als Basispolymer zur Wechselwirkung mit und Verpackung von DNA. Polylysin wurde mit verschiedenen Liganden (Transferrin, Insulin, Asialoorosomukoid oder Galactose) modifiziert, um modifizierte Protein-DNA-Komplexe auf Zelloberflächenrezeptoren zu bringen: Wu, G.Y. et al. in: J. Biol. Chem. (1987) 262:4429-4432, Cotten, M. et al. in: Proc. Natl. Acad. Sci. (USA) (1990) 87:4033-4037, Huckett, B. et al. in: Biochem. Pharmacol. (1990) 40:253-263, Plank, C. et al. in: Bioconjugate Chem. (1992), 533-539. Das Verfahren wurde durch Verwendung von inaktivierten Adenoviren dramatisch verbessert, um den Austritt von DNA aus Endosomen zu erleichtern, siehe: Wagner, E. et al. in: Proc. Natl. Acad. Sci. (USA) (1992) 89:6099-6103, Christiano, R. J. et al. in: Proc. Natl. Acad. Sci. (USA) (1993) 90:2122-2126. Der größte Nachteil der beschriebenen Ansätze betrifft die Unmöglichkeit, die Chemie der Proteinkonjugation zu steuern und solche Konjugate auf reproduzierbare Weise herzustellen.

Zur Zeit werden die besten Transfektionsergebnisse in vitro und in vivo mit Retroviren, Adenoviren und anderen erhalten, siehe beispielsweise: Kerr, W. G. und Mule J. J. in: J. Leucocyte Biol. (1994) 56:210-214, Hwu, P. und Rosenberg, S. A. in: Cancer Detect. Prevent. (1994) 18:43-50, Rosenfeld, M. A. et al. in: Cell (1992) 68:143-155. Trotzdem weist die Verwendung von viralen Vektoren verschiedene Aspekte auf wie die Notwendigkeit extensiver Zellkulturmanipulationen, geringe Titer für bestimmte Virussysteme und den Zelltropismus des Virus. Außerdem kann die Immunreaktivität gegen virale Vektoren Probleme bereiten. Insbesondere sind auch die Sicherheitsanforderungen in Zusammenhang mit der Verwendung von viralen Vektoren bisher noch nicht vollständig gelöst.

Liposomen wurden auch verwendet, um in vitro und in vivo DNA in Zellen einzuführen. Die erfolgreichsten Liposomsysteme verwenden kationische Lipide wie Dioleyloxypropyltrimethylammonium (DOTMA, das einen Reaktionspartner bildet in Kombination mit Phosphatidylethanolamin (PE)), Dioleoyloxypropyltrimethylammoniummethylsulfat (DOTAP), Dimethylaminoethancarbamoylcholesterol, Dioctadecylamidoglycylspermin, 2,3-Dioleyloxy-N-(2(spermincarboxamido)ethyl)-N,N-dimethyl-1-propanamin (DOSPA), das einen Reaktionspartner in Kombination mit PE bildet, siehe Felgner, P. L. in: Proc. Natl. Acad. Sci. (USA) (1987) 84:7413-7417, US-Patent Nr. 5208036, Leventis, R. und Silvius, J. R. in: Biochimica et Biophysica Acta (1990), 124-132, Gao, X. und Huang, L. in: Biochem. Biophys. Res. Commun. (1991) 179:280-285, Behr, J.-P. et al. in: Proc. Natl. Acad. Sci. (USA) (1989) 86:6982-6986. Weitere Lipidverbindungen sind in Chemical Abstracts, Vol. 114, No. 9 (1991), Abstract No. 76422 (JP 02 135 092 A) und ACS SYMP. Ser. (1986), Seiten 272, 273 beschrieben.

Der Vorteil der Verwendung der oben genannten Verbindungen liegt darin, daß das kationische Liposom einfach mit der DNA gemischt und der Zelle zugegeben wird. Die Transfektionseffizienz ist üblicherweise hoch im Vergleich zu anderen physikalischen Methoden der DNA-Übertragung. Außer zur Abgabe von DNA wurde eine spezifische Verbindung zur Abgabe von mRNA und Proteinen in kultivierte Zellen verwendet, siehe Malone, R. et al. in: Proc. Natl. Acad. Sci. (USA) (1989) 86:6077-6081 und Debs, R. et al. in: J. Biol. Chem. (1990) 265:10189-10193. Einige der oben genannten Verbindungen wurden zur Übertragung von Reportergenen oder therapeutisch nützlichen Genen in vivo verwendet, siehe Nabel, G. J. et al. in: Proc. Natl. Acad. Sci. (USA) (1993) 90:11307-11311, Zhu, N. et al. in: Science (1993) 261:209-211. Schließlich wurde ein DNA-Transfektionsprotokoll entwickelt, der das cyclische kationische Peptid Gramicidin S und PE verwendet, siehe Legendre, J.-Y. und Szoka, F. C. in: Proc. Natl. Acad. Sci. (USA) (1993) 90: 893-897. Das oben genannte System verwendet mit Vorteil die Bindungsfähigkeit von DNA und die Membrandestabilisierungseigenschaften von Gramicidin S.

Der größte Nachteil der kationischen Liposomen betrifft ihre relativ hohe Zytotoxizität. Außerdem sind die meisten der oben genannten Verbindungen in Gegenwart von Serum nicht wirksam oder zeigen eine stark reduzierte Wirksamkeit. Die meisten erfordern die Verwendung von PE, möglicherweise weil PE Intramembran-Lipid-Zwischenverbindungen bilden kann, die die Membranfusion erleichtern. Untersuchungen des der Transfektion zugrundeliegenden Mechanismus unter Verwendung von kationischen Lipiden wurden bisher noch nicht vollständig abgeschlossen. Es besteht daher ein Bedarf an einer weniger toxischen, nicht infektiösen und wirksameren Abgabe von biologischen Molekülen, insbesondere in das Zytoplasma und die Kerne von lebenden Zellen.

Die Erfindung stellt sich die Aufgabe, die geschilderten und weitere Nachteile des Standes der Technik zu vermeiden. Dabei sollen in erster Linie neue Lipidverbindungen zur Verfügung gestellt werden, die es erlauben, den Transport biologisch aktiver Stoffe oder Moleküle durch Membranen und damit in Zellen oder Zellorganellen zu verbessern.

Diese Aufgabe wird in erster Linie gelöst durch die Verbindungen gemäß Anspruch 1. Bevorzugte Verbindungen sind in den Unteransprüchen 2 bis 13 beansprucht. Erfindungsgemäße Folgeprodukte und Anwendungen der neuen Verbindungen ergeben sich aus den Ansprüchen 14 bis 26 (Komplexe), den Ansprüchen 27 und 28 (Verfahren zur Herstellung der Komplexe), Anspruch 29 (Liposom) und den Ansprüchen 30 bis 34 (Liposom-Formulierungen). Der Wortlaut sämtlicher Ansprüche wird hiermit durch Bezugnahme zum Inhalt dieser Beschreibung gemacht.

Die erfindungsgemäßen Verbindungen lassen sich durch die Formel I wie folgt darstellen: 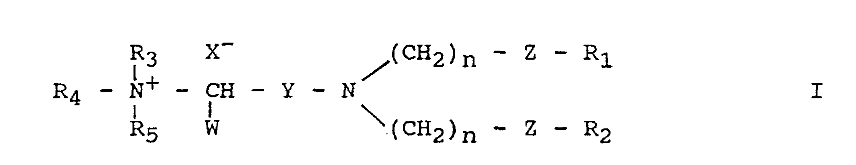 worin
- R₁ und R₂ gleich oder verschieden sind und eine Alkyl-, Alkenyl- oder Alkinylgruppe mit 6 bis 24 Kohlenstoffatomen sind;
- R₃ Wasserstoff, Alkyl oder Alkylamin mit 1 bis 8 Kohlenstoffatomen, oder eine Aminosäure, ein Aminosäurederivat, ein Peptid oder ein Peptidderivat sind;
- R₄ und R₅ gleich oder verschieden sind und Wasserstoff, Alkyl oder Alkylamin mit 1 bis 8 Kohlenstoffatomen sind;
- W ein Seitenkettenrest von einer basischen Aminosäure und ihren Derivaten, Peptiden oder Peptidderivaten ist;
- Y -CO-, -(CH₂)ₘCO-, -(CH₂-)ₘ, -(CHOHCH₂-)ₘ, worin m gleich 1 bis 20 ist, -CH₂-S-CH₂-, -CH₂-SO-CH₂, -CH₂-SO₂-CH₂-, -CH₂-SO₂- oder -SO₂- ist;
- Z eine Ester-, Ether- oder Amidbindung ist;
- n gleich 1 bis 8 ist; und
- X ein Anion ist.

Bei den erfindungsgemäßen Verbindungen handelt es sich um Lipide (Detergentien, Tenside), deren wesentliche Eigenschaften bereits beschrieben wurden. Die von der Formel I umfaßten Verbindungen können in Form ihrer optischen Isomere (R- oder S-Konfiguration) oder in Form deren Gemische vorliegen.

Gemäß Formel I sind die Lipidverbindungen in Form von aus Ionen gebildeten Salzen dargestellt. Dies ergibt sich daraus, daß auch an sich neutrale Verbindungen in (wäßriger) Lösung in Form von Ionen vorliegen. Selbstverständlich soll die Erfindung gegebenenfalls auch die entsprechenden neutralen Verbindungen umfassen. Verbindungen der Formel I können durch entsprechende Wahl der Substituenten weitere Ladungen tragen, beispielsweise im Substituenten W durch entsprechende Wahl des Seitenkettenrestes einer Aminosäure.

Zum besseren Verständnis der Ansprüche und der Beschreibung werden die folgenden Definitionen gemacht:
Alkyl bezeichnet ein voll gesättigtes verzweigtes oder unverzweigtes Kohlenstoffkettenradikal.
Alkenyl bezeichnet ein verzweigtes oder unverzweigtes ungesättigtes Kohlenstoffkettenradikal mit einer oder mehreren Doppelbindungen.
Alkinyl bezeichnet ein verzweigtes oder unverzweigtes ungesättigtes Kohlenstoffkettenradikal mit einer oder mehreren Dreifachbindungen.
Aminosäure bezeichnet eine monomere Einheit eines Peptids, Polypeptids oder Proteins. Die zwanzig Proteinaminosäuren (L-Isomere) sind: Alanin ("A"), Arginin ("R"), Asparagin ("N"), Aspariginsäure ("D"), Cystein ("C"), Glutamin ("Q"), Glutaminsäure ("E"), Glycin ("G"), Histidin ("H"), Isoleucin ("I"), Leucin ("L"), Lysin ("K"), Methionin ("M"), Phenylalanin ("F"), Prolin ("P"), Serin ("S"), Threonin ("T"), Tryptophan ("W"), Tyrosin ("Y") und Valin ("V"). Der hier verwendete Ausdruck Aminosäure umfaßt auch Analoge der Proteinaminosäuren, D-Isomere der Proteinaminosäuren, β-, γ- usw. Aminosäuren, unnatürliche Aminosäuren und ihre Analogen.

Biologisch aktive Substanz bezeichnet jedes Molekül oder Mischung oder Komplex von Molekülen,die in vitro und/oder in vivo eine biologische Wirkung erzielen, einschließlich Pharmazeutika, Arzneistoffe, Proteine, Steroide, Vitamine, Polyanionen, Nukleoside, Nukleotide, Polynukleotide usw.

In dieser Beschreibung bezeichnete Puffer umfassen: "Hepes", eine N-2-Hydroxyethylpiperazin-N'-2-ethansulfonsäure, die hier als Puffer bei pH um 7 verwendet wird; "PBS", eine Phosphatpuffersalzlösung mit 10 mM Phosphat und 0,9 Gew.-% NaCl, das hier als isotonischer physiologischer Puffer bei pH 7,4 verwendet wird; "Transfektionspuffer", 10 mM Hepes und 0,9 Gew.-% NaCl, das hier als Puffer bei pH um 7,4 verwendet wird; "Tris", Trishydroxymethylaminomethan, das hier ebenfalls als Puffer bei pH um 7 verwendet wird.

Komplexe oder Liposome, die auf Zellen gebracht werden (cell-targeted), besitzen weitere Moleküle z. B. auf ihrer Oberfläche, die fähig sind, eine Verbindung auf der Oberfläche der betreffenden Zelle zu erkennen. Zellerkennungsverbindungen umfassen: Liganden für Zelloberflächenrezeptoren, Antikörper für Zelloberflächenantigene usw.

Ladungsmaskierte (charge-masked) Komplexe oder Liposomen sind als positiv geladen zu verstehen, die eine Verbindung der Formel I umfassen und ein gebundenes Polymer, das z. B. die Oberfläche des Liposoms bedeckt. Ein Polymer kann kovalent mit einem Lipid verbunden sein, das das Liposom bildet oder auf der Oberfläche adsorbiert sein.

Ein Komplex ist definiert als das durch Mischen zweier oder mehrerer Komponenten hergestellte Produkt. Ein solcher Komplex ist gekennzeichnet durch eine nichtkovalente Wechselwirkung (ionisch, hydrophil, hydrophob usw.) zwischen zwei oder mehr Komponenten.

DNA stellt Desoxyribonukleinsäure dar, die unnatürliche Nukleotide umfassen kann. Die DNA kann einsträngig oder doppelsträngig sein.

Arzneistoff bezeichnet alle prophylaktischen oder therapeutischen Verbindungen, die zur Vorbeugung, Diagnose, Linderung, Behandlung oder Heilung von Krankheiten bei Mensch oder Tier verwendet werden.

Eine Liposomformulierung ist eine Zusammensetzung von Stoffen umfassend ein Liposom, das eingeschlossene Substanz zu diagnostischen, biologischen, therapeutischen oder anderen Verwendungszwecken enthält.

Ein gegebenenfalls vorhandenes Co-Lipid ist als eine Verbindung zu verstehen, die fähig ist, allein oder in Kombination mit anderen Lipidkomponenten ein stabiles Liposom zu bilden. Beispiele der wahlweisen Co-Lipide umfassen phospholipidartige Substanzen wie Lecithin, Phosphatidylcholin, Dioleylphosphatidylcholin (DOPC), Phosphatidylethanolamin (PE), Phosphatidylserin, Phosphatidylglycerin, Phosphatidylinositol, Sphigomyelin, Cephalin, Cardiolipin, Phosphatidsäure, Cerebroside, Dicetylphosphat usw., phosphorfreie Lipide wie Steroide und Terpene. Weitere phosphorfreie Lipide sind z. B. Stearylamin, Dodecylamin, Hexadecylamin, Acetylpalmitat, Glycerinricinoleat, Hexadecylstearat, Isopropylmyristat, Dioctadecylammoniumbromid, amphotere Polymere, Triethanolaminlaurylsulfat, kationische Lipide wie sie zuvor beschrieben wurden und dergleichen Verbindungen.

Ein pharmazeutisch akzeptables Ion ist ein Ion, das selbst nicht toxisch ist.

Ein Polyanion ist eine polymere Struktur, wo mehr als eine Einheit des Polymers eine negative Ladung trägt und die Nettoladung des Polymers negativ ist.

Ein Polykation ist eine polymere Struktur, wo mehr als eine Einheit des Polymers eine positive Ladung trägt und die Nettoladung des Polymers positiv ist.

Ein Polynukleotid ist z. B. DNA oder RNA, die mehr als ein Nukleotid enthalten. Polynukleotide sollen auch cyclische Polynukleotide und unnatürliche Nukleotide umfassen und sie können nach chemischen Verfahren hergestellt werden oder durch Verwendung der Rekombinantentechnik oder durch beides.

Ein Polypeptid ist als eine Reihe von zwei oder mehr Aminosäuren zu verstehen, die über kovalente Bindung verknüpft sind.

RNA stellt Ribonukleinsäure dar, die auch unnatürliche Nukleotide umfassen kann. Die RNA kann einsträngig oder doppelsträngig sein.

Bei den bereits beschriebenen Verbindungen gemäß Formel I ist es bevorzugt, wenn Y Carbonyl, d. h. -CO- ist. Damit erfolgt die Verknüpfung nach Art einer Peptidbindung -CO-N〈.

Die Gruppe Z umfaßt bei den Lipidverbindungen gemäß Formel I vorzugsweise eine Esterbindung, d. h. die Gruppe -O-CO-.

Bei weiteren bevorzugten Ausführungsformen sind R₁ und R₂ eine Alkyl- oder Alkenylgruppe mit 10 bis 20, vorzugsweise 12 bis 18 Kohlenstoffatomen. Dabei sind die Reste R₁ und R₂ vorzugsweise gleich. Von den beschriebenen bevorzugten Gruppen sind wiederum Alkenylgruppen bevorzugt. Dementsprechend handelt es sich bei den Resten R₁ und R₂ neben Palmityl- oder Stearylgruppen, vorzugsweise um die Oleylgruppe oder um Reste der Linol- oder Linolensäure.

Bei der Formel I ist n vorzugsweise gleich 2. Weiter handelt es sich bei der Gruppe W vorzugsweise um einen Seitenkettenrest von Lysin oder Ornithin.

Bei den Resten R₃, R₄ und R₅ kann es sich um Alkylgruppen mit 1 bis 8 Kohlenstoffatomen, insbesondere um die Methylgruppe handeln. Dabei sind die drei Reste R₃, R₄ und R₅ vorzugsweise gleich. Bei besonders bevorzugten Ausführungsformen sind R₃, R₄ und R₅ Wasserstoff.

Als Gegenion X können im Prinzip alle denkbaren Anionen verwendet werden, wobei für die meisten Anwendungen der Erfindung pharmazeutisch annehmbare Anionen bevorzugt sind. Vorzugsweise kann es sich bei X um ein Halogenidanion, insbesondere um das Chloridanion handeln.

Bevorzugte Verbindungen gemäß Formel I ergeben sich aus Anspruch 12, auf den hier ausdrücklich Bezug genommen wird. Eine besonders bevorzugte Verbindung ist L-Lysin-bis-(O,O'cis-9-octadecenoyl-β-hydroxyethyl)amid-dihydrochlorid oder ein optisches Isomer davon.

Verbindungen, die unter die Definition der Formel I fallen und erfindungsgemäß bevorzugt sind, lassen sich auch dadurch darstellen, daß man die entsprechenden Verbindungen durch die folgende Verknüpfung darstellt:

Aminosäure oder Peptid ----- Dialkanolamin ----- Fettsäure oder Fettalkohol

Die auf diese Weise darstellbaren Verbindungen sind für die im folgenden noch zu diskutierenden Anwendungen besonders geeignet. Insbesondere zeichnen sie sich durch eine weitaus geringere Toxizität als bisher bekannte Lipidverbindungen, die für ähnliche Anwendungen eingesetzt werden, aus.

Neben den beschriebenen Lipidverbindungen umfaßt die Erfindung Komplexe, die mit Hilfe dieser neuen Verbindung herstellbar sind. Dabei soll "Komplex" im Sinne der obigen Definition verstanden werden. Es muß sich nicht notwendigerweise um ein vollständig ausgebildetes Liposom handeln. Da allerdings die genauen Mechanismen bei der Komplexbildung nicht bekannt sind, soll der Fall, daß sich aus dem Lipid zunächst Liposome bilden und diese dann mit Polyanionen, Polykationen oder deren Komplexe komplexieren, nach der Erfindung nicht ausgeschlossen sein.

Die Eigenschaften der erfindungsgemäßen Komplexe sind in den Ansprüchen 14 bis 19 bzw. 20 bis 26 beansprucht und beschrieben. Auf diese Ansprüche wird ausdrücklich Bezug genommen. Die Komplexbildung ist dabei im wesentlichen darauf zurückzuführen, daß die erfindungsgemäßen Verbindungen positiv geladen sind.

Das Verhältnis zwischen Lipidverbindung und den anderen Bestandteilen des Komplexes, d. h. bei den (binären) Polyanion-Lipid-Komplexen das Verhältnis zwischen Polyanion und Lipidverbindung kann je nach gewünschter Anwendung stark variiert werden. Vorzugsweise ist das Verhältnis zugunsten der Lipidverbindung größer als 1 : 1 (bezüglich der Ladung), so daß für den Komplex eine positive Gesamt- bzw. Nettoladung resultiert, um auf einfache Weise mit der negativ geladenen Oberfläche von biologischen Membranen wechselwirken zu können. Gegebenenfalls wird ein besonders vorteilhaftes Verhältnis experimentell bestimmt. So kann beispielsweise bei einer DNA-Transfektion dasjenige Verhältnis von DNA und Lipidverbindung experimentell ermittelt werden, das zu einer optimalen Expression der transfektierten DNA führt.

Grundsätzlich können die erfindungsgemäßen Komplexe mit allen Molekülen ausgeführt werden, die eine negative Ladung aufweisen. Bevorzugt sind solche Komplexe, bei denen ein Polynukleotid als Polyanion eingesetzt wird.

Bei den in den Ansprüchen 20 bis 26 dargestellten ternären Komplexen ist es bevorzugt, wenn es sich bei dem Polykation um ein Polypeptid handelt. Grundsätzlich ist es jedoch möglich, auch neutrale Polypeptide zur Bildung ternärer Polynukleotid-Polypeptid-Lipid-Komplexe einzusetzen, die ebenfalls von der Erfindung umfaßt sein sollen.

Überraschenderweise wurde durch die Ausbildung ternärer Komplexe festgestellt, daß beim Einsatz von Polypeptiden beispielsweise die Transfektion von Polynukleotiden gegenüber dem Einsatz von Polynukleotid-Lipid-Komplexen weiter beträchtlich erhöht werden kann. Dabei können die in Anspruch 25, auf den hier ausdrücklich Bezug genommen wird, genannten Peptidsequenzen bevorzugt eingesetzt werden. Dabei repräsentiert die erste Sequenz den C-terminalen Part des Kernproteins des Hepatitis-B-Virus (HBV). Obwohl eine abschließende wissenschaftliche Erklärung zur Zeit noch nicht gegeben werden kann, scheint ein Zusammenhang des positiven Einflusses dieser Peptidsequenzen bei den ternären Komplexen und dem vergleichsweise hohen Anteil an basischen Aminosäuren in den Sequenzen möglich zu sein.

Gegebenenfalls können die beschriebenen Komplexe für bestimmte Anwendungen ladungsmaskiert (charge-masked) oder zur Wechseiwirkung mit bestimmten Zellen oder Zellorganellen ausgebildet (cell-targeted) sein.

Weiter umfaßt die Erfindung die in den Ansprüchen 27 und 28 beschriebenen Verfahren zur Herstellung der erfindungsgemäßen Komplexe. Dabei erfolgt diese Herstellung und das damit verbundene in Kontakt bringen durch übliche Verfahren, die dem Fachmann bekannt sind. So werden beispielsweise zur Herstellung von binären Komplexen Pufferlösungen, die das Polyanion bzw. die Lipidverbindung enthalten, vereinigt. Entsprechendes gilt für die Herstellung von ternären Komplexen, wobei zunächst in einer Pufferlösung ein Polyanion-Polykation-Komplex dargestellt und diese Pufferlösung anschließend mit einer die Lipidverbindung enthaltenden Pufferlösung vereinigt wird.

Weiter umfaßt die Erfindung Liposome, die mit mindestens einer erfindungsgemäßen Lipidverbindung herstellbar oder hergestellt sind. Die Herstellung der Liposomen erfolgt dabei in üblicher aus dem Stand der Technik bekannter Weise.

Weiter umfaßt die Erfindung Liposom-Formulierungen in wäßriger Lösung, die mindestens eine biologisch aktive Substanz (Stoff, Molekül usw.) und eine Lipidkomponente umfassen. Dabei enthält die Lipidkomponente mindestens eine der erfindungsgemäßen Lipidverbindungen. Neben der biologisch aktiven Substanz und der Lipidkomponente enthält die Liposom-Formulierung übliche Lösungsbestandteile der wäßrigen Lösung, wobei es sich um eine Lösung in reinem Wasser oder vorzugsweise um übliche Pufferlösungen handeln kann.

Wie aus der Formulierung von Anspruch 30 hervorgeht, kann als Lipidkomponente eine einzelne Verbindung der Formel I oder ein Gemisch solcher Verbindungen eingesetzt werden. Außerdem ist es möglich, die erfindungsgemäße Verbindung bzw. Verbindungen mit weiteren Co-Lipiden wie sie bereits definiert sind, zum Beispiel PE, POPE in Mischung einzusetzen.

Solange eine Liposombildung möglich ist, ist die Menge an biologisch aktiver Substanz grundsätzlich nicht kritisch. Üblicherweise wird die Substanz in Mengen bis zu 10 Gew.-% in der Liposom-Formulierung vorhanden sein. Als Untergrenze ist beispielsweise ein Wert von 0,01 Gew.-% zu nennen. Ein Mengenbereich von 1 bis 5 Gew.-% an biologisch aktiver Substanz ist bevorzugt.

Die erfindungsgemäße Verbindung kann vorzugsweise 1 % bis 100 % der Lipidkomponente ausmachen. Bei bevorzugten Ausführungen der Liposom-Formulierung sind ein oder mehrere Co-Lipide vorhanden, wobei diese Co-Lipide vorzugsweise 30 bis 70 % der Lipidkomponente ausmachen.

Handelt es sich bei der biologisch aktiven Substanz um einen Arzneistoff, so wird dessen Menge üblicherweise nach der gewünschten Therapie ausgewählt. Dabei sind vorzugsweise 1 bis 5 Gew.-% des Arzneistoffes in der Liposom-Formulierung vorhanden. Gegebenenfalls können beim Einsatz von Arzneistoffen als biologisch aktiver Substanz pharmazeutisch akzeptable Exzipienten in der Liposom-Formulierung vorhanden sein.

Wie bei den erfindungsgemäßen Komplexen bereits erwähnt, können auch die erfindungsgemäßen Liposomen oder Liposom-Formulierungen gegebenenfalls ladungsmaskiert oder zur Wechselwirkung mit bestimmten Zellen (cell-targeted) ausgebildet sein.

Schließlich sind Verfahren zum Transport von Polyanionen oder Polykationen bzw. zum Transport von biologisch aktiven Substanzen allgemein durch biologische Membranen, insbesondere zum Einbringen in Zellen oder Zellorganellen, beschrieben. Für die solche Verfahren können entweder die erfindungsgemäßen Verbindungen selbst oder Zusammensetzungen, die solche Verbindungen enthalten, eingesetzt werden.

Bei Durchführung der Verfahren durch Inkubieren in vivo können zusätzliche Stabilisatoren wie beispielsweise Polyethylenglykol vorhanden sein.

Weitere Einzelheiten der erfindungsgemäßen Verfahren werden noch beschrieben.

Die Verbindungen und andere Teile der vorliegenden Erfindung bringen verschiedene Vorteile. Einer der Vorteile der hier beschriebenen Verbindungen ist, daß sie bis zu 100 % Einschluß von polyanionischen Substanzen mit einem zweckmäßigen Protokoll erlauben. Andererseits führt die Inkubation von positiv geladenen Komplexen mit negativ geladenen Zelloberflächen zu einer schnellen und besseren Aufnahme insbesondere von polyanionischen Substanzen und anderen biologisch aktiven Verbindungen im allgemeinen. Das letztere erlaubt das Einführen von komplexierten bzw. eingeschlossenen polyanionischen Substanzen wie beispielsweise DNA in einem Maße wie es bisher bei diesen Zellen nicht bekannt ist.

Die speziellen Vorteile der hier offenbarten Verbindungen sind wie folgt. Zunächst stellen diese Verbindungen die neuen liposombildenden Lipide dar. Die Geometrie der beiden aliphatischen Ketten in den Verbindungen der Formel I erlaubt ihre Organisation in stabile Doppelschichtstrukturen. Der polare Kopf (z. B. Aminosäure) kann in Abhängigkeit von der Anwendung variiert sein. Dies erlaubt zum Beispiel leicht das Einführen verschiedener Modifikationen an der Aminogruppe, der Seitenkette und der Bindung. Die Zytotoxizität der meisten der hier offenbarten kationischen Verbindungen sind im Vergleich zu den zuvor bei anderen kationischen Amphiphilen berichteten günstig. Alle bei den Verbindungen der Formel I dargestellten Bindungen können leicht in der Zelle hydrolysiert werden, was zur Ausbildung nicht toxischer Verbindungen führt.

Die positiv geladenen Lipide der Formel I besitzen ferner, im Gegensatz zu anderen kationischen Lipiden aus dem Stand der Technik, eine bessere Transfektionseffizienz in Gegenwart von Serum. Die Fähigkeit, Zellen in der ständigen Gegenwart von Serum zu transfektieren ist aus verschiedenen Gründen vorteilhaft: die Transfektion verläuft leichter und ist weniger zeitaufwendig, die Anforderungen an Medien und Serum sind geringer, den Zellen wird kein Serum entzogen, was Zellfunktionen und Lebensfähigkeit beeinträchtigen könnte.

Der zweite spezifische Vorteil der hier offenbarten Technik leitet sich von dem neuen Verfahren zum Einbringen von Polynukleotid (insbesondere DNA) in ternäre Komplexe ab. Dieser Komplex ist insbesondere gebildet aus positiv geladenen Lipiden der Formel I und einem Komplex aus Polynukleotid und kationischem Polypeptid. Gemäß dem Verfahren wird zuerst der erste Komplex aus Polynukleotid-kationischem Polypeptid gebildet, der im nächsten Schritt mit einem positiv geladenen Lipid der Formel I komplexiert wird. Ein exaktes Abstimmen der Zusammensetzung bestimmt die biologische Aktivität des schließlich erhaltenen Komplexes.

Der Vorteil dieser Vorgehensweise gegenüber den aus dem Stand der Technik bekannten Transfektionstechniken liegt beispielsweise darin, daß die neue Methode zu einer bis zu 300fach höheren Transfektionseffizienz führt. Darüber hinaus kann bei Anwendung der neuen Methode ein Nachweis der Transfektion (z. B. Nachweis des exprimierten Proteins) nach weniger als zwei Stunden nach Beginn der Transfektion leicht registriert werden. Außerdem erlaubt das neue Verfahren ein Arbeiten im "Mikromaßstab" zur Transfektion von Zellen (beispielsweise 96-Loch-Format), was für das Screening einer großen Zahl von Proben wünschenswert ist und die Automatisierung des gesamten Verfahrens ermöglicht.

Die Verbindungen der Formel I sind insbesondere nützlich bei der Herstellung von Liposomen, können aber auch in anderen Fällen verwendet werden, wo kationische Lipide Anwendung finden. Zum Beispiel können sie in industriellen Anwendungen verwendet werden. Von besonderem Interesse ist die Verwendung dieser Verbindungen in Zusammenhang mit kationischen Lipiden, die für pharmazeutische Formulierungen (Cremes, Pasten, Gele, kolloide Dispersionen und dergleichen) und/oder kosmetische Zusammensetzungen (Makeups, Lippenstifte, Nagellacke, Körperlotionen, Feuchtigkeitscremes, Shampoos und dergleichen) akzeptabel sind.

Formulierungen umfassend die Verbindungen der Formel I sind vorteilhaft zur Erzielung einer gewünschten intrazellulären Abgabe von biologisch aktiven Substanzen wie Polynukleotiden, Peptiden, Proteinen, Steroiden und anderen natürlichen oder synthetischen Verbindungen. Die intrazelluläre Abgabe kann in das Zytoplasma, in den Kern oder beides erfolgen. Eine solche intrazelluläre Abgabe kann in Gewebekulturen (in vitro) erreicht werden und kann beispielsweise verwendet werden, um Zellen mit gewünschten Polynukleotiden (z. B. DNA) zu transfektieren oder um Proteine oder dergleichen abzugeben.

Formulierungen umfassend die Verbindungen der Formel I können auch zur Therapie ex vivo verwendet werden, wo aus den Organismus isolierte Zellen in vitro transfektiert werden und dann in den Organismus implantiert werden. Ein Beispiel für diese Anwendung ist das Transfektieren von Knochenmarkszellen.

Eine intrazelluläre Abgabe kann auch im ganzen Organismus (in vivo) erreicht werden und kann daher bei verschiedenen Anwendungen nützlich sein wie Gentherapie, Antisensibilisierungsund Antigentherapie. Intrazelluläre Abgabe in vivo kann auch zur DNA-Impfung verwendet werden mit dem Ziel, eine Immunreaktion (humoral und/oder zellulär) auf das gewünschte Protein zu induzieren.

Intrazelluläre Abgabe unter Verwendung der Verbindungen der Formel I kann auch nützlich sein zur Abgabe von Antikrebsund Antivirusverbindungen, Antibiotika und dergleichen.

Eine Zellselektivität kann erreicht werden durch Einbringen von Zellerkennungsverbindungen, z. B. auf der Oberfläche des Vesikels wie Antikörper, Liganden für Zelloberflächenrezeptoren und dergleichen. Eine erhöhte Stabilität und weitere Selektivität kann erreicht werden, z. B. durch Überziehen des Liposomvesikels mit einer geeigneten ladungsmaskierten natürlichen oder synthetischen Verbindung wie Polymeren und neutralen oder negativ geladenen Lipiden.

Liposomvesikel umfassend Verbindungen der Formel I können zur Auslösung einer spezifischen Immunreaktion auf ein bestimmtes Antigen verwendet werden, das in das Liposom eingebracht ist. Weitere Komponenten wie N-Palmitoyl-S-(2,3-bis(palmitoyloxy)-(2RS)-propyl)-R-cystein (Pam₃Cys) oder N-Acetyl-myramyl-L-threonyl-D-isoglutamin (MDP) und Derivate davon können besonders nützlich sein.

Verbindungen der Formel I sind von Interesse für die Einführung einer lipophilen Gruppe in Polymere und insbesondere in Peptide, um ihre Aufnahme durch eine Zelle zu erhöhen oder um ihre Inkorporation in die lipidhaltigen Vesikel und dergleichen zu erhöhen. Aktivierte Verbindungen der Formel I können auch zur Modifizierung von Proteinen verwendet werden.

Weitere Einzelheiten der bisher vorgestellten Teile der Erfindung ergeben sich durch die folgende Beschreibung von Herstellungsverfahren, Lipidverbindungen und Beispielen für deren Verwendung. Auf die beigefügten Figuren wird jeweils bei der Beschreibung des jeweiligen Beispiels verwiesen.

### Verfahren zur Darstellung ausgewählter Verbindungen der Formel I

Unter Verwendung bekannter Techniken kann der Fachmann die hier angegebenen bevorzugten Polypeptidaminosäuresequenzen leicht durch Zusätze, Elimination oder Substitution, Erhöhung oder Verringerung abwandeln oder einfach eine andere Sequenzkodierung für verschiedene kationische Polypeptide verwenden. Es ist jedoch anzumerken, daß solche Variationen im Rahmen dieser Erfindung liegen. Es ist ferner anzumerken, daß die nachfolgend angeführten Beispiele nur zum Zwecke der Erläuterung angegeben sind und nicht als Einschränkung dieser Erfindung in irgendeiner Weise zu verstehen sind.

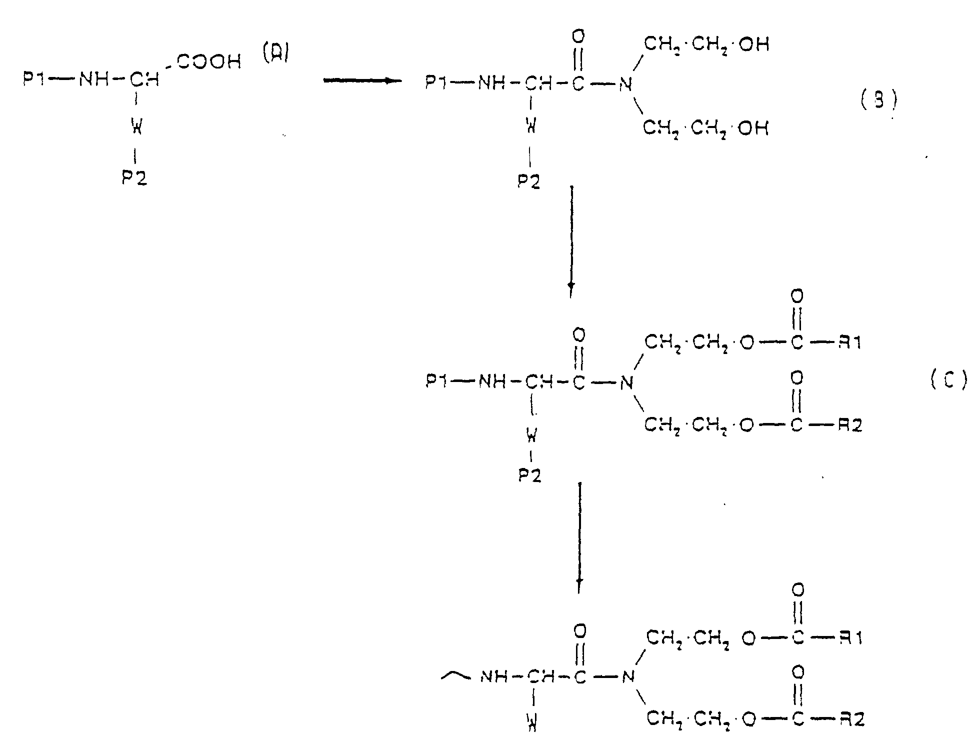

Dieses Reaktionsschema ist für die Synthese von bevorzugten Verbindungen der Formel I anwendbar, worin Z eine Esterbindung und n gleich 2 ist. In diesem Reaktionsschema sind P¹ und P² Schutzgruppen und sie sind gleich oder unterschiedlich, W ist eine Seitenkette einer Aminosäure, R¹ und R² sind gleich und sind Alkyl oder Alkenyl mit 6 bis 24 Kohlenstoffatomen.

Verbindungen der Formel (A) sind im Handel in optisch reiner Form erhältlich. Zur Bildung der Verbindungen der Formel (B) wird die Carboxylgruppe der Aminosäure der Formel (A) mit einem geeigneten Reagens aktiviert und dann mit der Iminogruppe von Diethanolamin umgesetzt. Verfahren zur Aktivierung von Aminosäuren sind im Stand der Technik bekannt. Beispielsweise kann das Verfahren mit Dicyclohexylcarbodiimid/N-Hydroxysuccinimid zur epimerisierungslosen Amidierung geschützter Aminosäuren angewendet werden.

Das Amid der Formel (B) wird durch Auflösen der Aminosäure von Formel (A) und einem zuvor gebildeten Salz von N-Hydroxysuccinimid und Diethanolamin in einem geeigneten polaren Lösungsmittel wie Dimethylformamid hergestellt. Die Mischung wird auf 0 °C gekühlt und dann eine Lösung von Dicyclohexylcarbodiimid zugegeben. Die Umsetzung wird durch Rühren der Lösung eine Stunde lang bei 0 °C und acht Stunden lang bei Raumtemperatur durchgeführt. Das erhaltene Amid der Formel (B) wird dann nach üblichen Abtrennverfahren gewonnen.

Um die Bildung der Verbindungen der Formel (C) zu erreichen, wird das Amid der Formel (B) in einem geeigneten Lösemittel (z. B. Dichlormethan) aufgelöst. Dazu wird ein geeignetes tertiäres Amin wie beispielsweise Triethylamin in molarem Überschuß zugegeben. Die Mischung wird auf ungefähr 0 °C gekühlt. Das Alkylierungsmittel mit gewünschter Kettenlänge und ungesättigten Gruppen wird in einem molaren Überschuß zugegeben, bevorzugt in ungefähr der 3- bis 4fachen Menge des Amids der Formel (B). Es kann beispielsweise Oleoylchlorid verwendet werden, um die Addition von 9-Octadecenoylgrupppen zu erreichen. Diese Mischung wird dann ungefähr 3 Stunden unter N₂-Atmosphäre bei Raumtemperatur gerührt. Dann wird das Produkt der Formel (C) extrahiert.

Die Verbindung der Formel (C) wird dann in geeigneter Weise von ihren Schutzgruppen befreit, wobei die Verfahrensweise von den verwendeten Schutzgrupppen abhängt, extrahiert und durch Chromatographie gereinigt.

### Beispiel 1

### Herstellung von L-Lysin-bis-(O,O'-oleoyl-β-hydroxyethyl)amiddihydrochlorid (1)

Boc-Lys(Boc)-OH *DCHA (2,63 g, 5 mmol) werden in Ethylacetat suspendiert und unter Verwendung von eiskalter 2 M H₂SO₄ in die freie Säure überführt. Boc-Lys(Boc)-OH in Ethylacetat wird mit MgSO₄ getrocknet und zur Trockene verdampft. Das verbliebene Öl wird in ungefähr 15 ml Dimethylformamid (DMF) suspendiert, das 1,1 g (5 mmol) eines zuvor gebildeten Salzes von N-Hydroxysuccinimid und Diethanolamin (HOSu*N(EtOH)₂) enthält, und wird mit Eiswasser auf 0 °C gekühlt. Unter Rühren wird eine Lösung von 1,13 g (5,5 mmol) Dicyclohexylcarbodiimid (DCC) in 5 ml DMF der Mischung zugegeben, die eine Stunde bei 0 °C gerührt wird und dann nochmals 8 Stunden bei Raumtemperatur. Die Mischung wird im Vakuum zur Trockene konzentriert. Nach Zugabe von ungefähr 50 ml Ethylacetat zum Rückstand fällt der größte Teil des Dicyclohexylharnstoffes (DCU) aus und wird abfiltriert. Die Ethylacetatphase wird mit wäßrigen Lösungen von NaHCO₃ (5 %) und Citronensäure (5%) in 0,1 M NaCl gewaschen, mit MgSO₄ getrocknet und zur Trockene eingedampft, so daß 1,7 g des Rohprodukts als farbloses Öl erhalten werden.

Eine Lösung von 1,7 g (3,92 mmol) Boc-Lys(Boc)-N(EtOH)₂, 1,64 ml Triethylamin und 3,93 ml Oleoylchlorid (jeweils 11,7 mmol) in 50 ml Dichlormethan (DCM) werden 30 min lang bei 0 °C und 4 Stunden unter N₂-Atmosphäre im dunkeln bei Raumtemperatur gerührt. Die Mischung wird mit Methanol abgeschreckt, im Vakuum konzentriert, in Hexan aufgelöst und dreimal mit 0,1 M KOH in Methanol/Wasser (1 : 1 Vol.) bei 0 °C, dann einmal mit 0,1 M wäßriger NaCl gewaschen. Die Hexanphase wird im Vakuum konzentriert und der Rückstand mit 50 ml einer Mischung aus Trifluoressigsäure (TFA)/DCM (1 : 1 Vol.) 40 min bei Raumtemperatur behandelt. Die Mischung wird wiederholt mit Toluol gemischt und im Vakuum aufkonzentriert und dann in Chloroform auf eine Säule mit Silica Gel 60 aufgegeben. Die Säule wird mit einem aufsteigenden Gradienten von Methanol in Chloroform eluiert und die reine Verbindung (2,3 g, 62 % Ausbeute) bei ungefähr 20 % Methanol eluiert. Das Hydrochlorid wird durch Auflösen des Produktes in trockenem Ethylacetat, das mit HCl-Gas gesättigt ist, und Eindampfen zur Trockene hergestellt. (Analyse: ES-MS: bestimmte Molmasse 762 (berechnet 762); TLC: Rf = 0,69 in Ethylacetat:Essigsäure:Wasser = (4:1:1); HPLC: Rt = 14,19 min, Säule Nucleosil C2 4,0x100 mm, Gradient 30 - 90 % Acetonitril in 0,1 % TFA in Wasser in 20 min).

In gleicher Weise, aber durch Substitution des geeigneten Ausgangsmaterials werden die folgenden Verbindungen hergestellt:
L-Lysin-bis-(O,O'-palmitoyl-β-hydroxyethyl)amid-dihydrochlorid (2)
L-Lysin-bis-(O,O'-myristoyl-β-hydroxyethyl)amid-dihydrochlorid (3)
L-Ornithin-bis-(O,O'-myristoyl-β-hydroxyethyl)amid-dihydrochlorid (4)
L-Ornithin-bis-(O,O'-oleoyl-β-hydroxyethyl)amid-dihydrochlorid (5)
L-Ornithin-bis-(O,O'-palmitoyl-β-hydroxyethyl)amid-dihydrochlorid (6)
L-Arginin-bis-(O,O'-oleoyl-β-hydroxyethyl)amid-dihydrochlorid (7)
L-Arginin-bis-(O,O'-palmitoyl-β-hydroxyethyl)amid-dihydrochlorid (8)
L-Serin-bis-(O,O'-oleoyl-β-hydroxyethyl)amid-dihydrochlorid (9)
Glycin-bis-(O,O'-palmitoyl-β-hydroxyethyl)amid-dihydrochlorid (10)
Sarcosin-bis-(O,O'-palmitoyl-β-hydroxyethyl)amid-dihydrochlorid (11)
L-Histidin-bis-(O,O'-palmitoyl-β-hydroxyethyl)amid-dihydrochlorid (12)
L-Glutamin-bis-(O,O'-palmitoyl-β-hydroxyethyl)amid-dihydrochlorid (13).

### Beispiel 2

### Synthese von N-α-tert-Butoxycarbonyl-L-Asparaginsäure-α-N'bis-(O,O'-palmitoyl-β-hydroxyethyl)amid (Boc-Asp-N(EtOPalm)₂ (14)) und N-α-Fluorenylmethyloxycarbonyl-L-Asparaginsäure-α-N'-bis-(O,O'-palmitoyl-β-hydroxyethyl)amid (Fmoc-Asp-N(EtOPalm)₂ (15))

Die Verbindung Boc-Asp(OBzl)-N(EtOH)₂ wird auf dieselbe Weise hergestellt wie oben für die Herstellung der Verbindung Boc-Lys(Boc)-N(EtOH)₂ beschrieben. Die Verbindung Boc-Asp(OBzl)-N(EtOPalm)₂ wird mit wenigen Modifikationen in derselben Weise hergestellt wie die Verbindung Boc-Lys(Boc)-N(EtOOL)₂. Die Reaktion läuft über Nacht, die Mischung wird mit Ethylacetat verdünnt und dann in eine gesättigte Lösung von NaHCO₃ gegossen, um restliches Palmitoylchlorid zu zerstören und Natriumpalmitat auszufällen. Der Niederschlag wird abfiltriert und die organische Phase zur Trockene evakuiert und in heißem Methanol aufgelöst. Das Produkt wird in kaltem Methanol ausgefällt und durch Filtration in einer Ausbeute von 55 % gewonnen (TLC: Rf = 0,64 in Chloroform : Methanol = 100 : 2). Die Benzylschutzgruppe wird vom Produkt (1,5 g) durch Behandeln mit NH₄COOH (0,65 g) in Gegenwart von frischem Pd-Schwarz (ungefähr 0,1 g) in 15 ml DMF über Nacht entfernt. Pd-Schwarz wird abfiltriert und die Mischung im Vakuum verdampft. Der Rückstand wird aus Methanol/Wasser ausgefällt, so daß das gewünschte Produkt Boc-Asp-N(EtOPalm)₂ (14) in einer Ausbeute von 85 % erhalten wird. (Analyse: ES-MS: bestimmte Molmasse 796,5 (berechnet 796); TLC: Rf = 0,4 in Chloroform : Ethylacetat : Methanol = 9 : 3 : 1).

0,5 g (0,62 mmol) Boc-Asp-N(EtOPalm)₂ werden mit einer Mischung von TFA/DCM = 1 : 1 30 min lang bei Raumtemperatur behandelt. Die Mischung wird wiederholt mit Toluol verdampft. Der Rückstand wird in 10 ml DMF aufgelöst, mit Diisopropylethylamin (DIPEA) neutralisiert und zwei Stunden lang mit 1,2 Equivalenten 9-Fluorenylmethylsuccinimidylcarbonat (Fmoc-OSu) umgesetzt. Die Reaktionsmischung wird verdampft und in Methanol wieder aufgenommen. Die gewünschte Verbindung Fmoc-Asp-N(EtOPalm)₂ (15) wird ausgefällt, so daß 0,44 g der Verbindung in einer Ausbeute von 77 % erhalten werden (Analyse: TLC: Rf = 0,4 in Chloroform:Ethylacetat:Methanol = 9:3:1).

Unter Verwendung ähnlicher Verfahrensweisen, aber Substitution des geeigneten Ausgangsmaterials, können die folgenden Verbindungen hergestellt werden:
N-α-tert-Butoxycarbonyl-L-glutaminsäure-γ-N'-bis-(O,O'palmitoyl-β-hydroxyethyl)amid Boc-Glu(N(EtOPalm)₂)-OH (16) N-α-Fluorenylmethyloxycarbonyl-L-glutaminsäure-γ-N'-bis-(O,O'-palmitoyl-β-hydroxyethyl)amid Fmoc-Glu(N(EtOPalm)₂)-OH (17)
N-α-tert-Butoxycarbonyl-L-asparaginsäure-β-N'-bis-(O,O'palmitoyl-β-hydroxyethyl)amid Boc-Asp(N(EtOPalm)₂)-OH (18)

Außerdem können die Zwischenprodukte ihrer Synthese oder der oben beschriebenen Synthese zur Herstellung von Liposomen verwendet werden:
L-Glutaminsäure-γ-N'-bis-(O,O'-palmitoyl-β-hydroxyethyl)amid H-Glu(N(EtOPalm)₂)-OH (19)
L-Asparaginsäure-β-N'-bis-(O,O'-palmitoyl-β-hydroxyethyl)amid H-Asp(N(EtOPalm)₂)-OH (20)
L-Asparaginsäure-α-N'-bis-(O,O'-palmitoyl-β-hydroxyethyl)amid H-Asp(N(EtOPalm)₂) (21)

### Herstellung und Eigenschaften eines Komplexes aus DNA und Verbindung (1)

### Beispiel 3

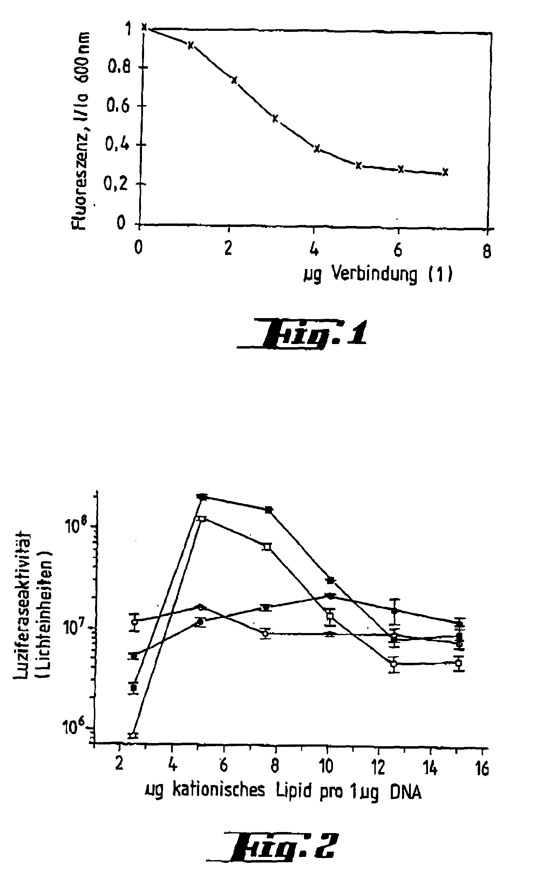

Fluoreszenz-Untersuchungen. Fluoreszenzmessungen werden mit einem Aminco SPF-1000Sc Spektrophotometer durchgeführt, wobei eine 1 cm Lichtwegzelle mit einer Schlitzweite zur Anregung und Emission von 10 nm verwendet werden. Die Bindung von (1) an Nukleinsäuren wird aus der Verdrängung des Ethidiumbromids abgeleitet, das bei Einfügung zwischen die DNA-Basenpaare als Fluoreszenzsonde wirkt. Die Fluoreszenz wird unmittelbar nach der Zugabe steigender Mengen von (1) zu mit Ethidiumbromid (5 µg/ml Kalbsthymus-DNA, 8x10⁻⁶ M bp (base pairs); Ethidiumbromid 1 : 50 bp in 10 mM Hepes, 150 mM NaCl, pH 7,4) komplexierter DNA gemessen. Die Ethidiumbromidverdrängung wird durch die Abnahme der Ethidiumbromidfluoreszenz (Anregung = 540 nm, Emission = 600 nm) verfolgt, die auftritt, wenn es von der DNA freigesetzt wird (Fig. 1). Die Fluoreszenzintensität nimmt mit zunehmender Konzentration des kationischen Lipids graduell ab. Dies zeigt, daß Kompaktierung und/oder Aggregation der DNA eintritt. Das Verhältnis zwischen (1) und DNA, bei dem die Fluoreszenzlöschung sich nicht mehr ändert, entspricht ungefähr 6 : 1 (Gew./Gew.). Das erhaltene Verhältnis entspricht einem Ladungsverhältnis von ungefähr 3,5 : 1.

### Transfektion von Zellen

### Beispiel 4

### Zellkulturen und Plasmide

HeLa (CCL2, humanes Epithelkarizom), COS-7 (CRL 1651, Niere, SV-40 transformiert, Afrikanischer grüner Affe), HT-29 (HTB 38, humanes Kolon-Adenokarzinom), CV-1 (CCL 70, Niere, Afrikanischer grüner Affe), 818-4 (humanes Pankreas-Adenokarzinom), H4IIE (CRL 1548, Hepatom, Ratte), K562 (CCL 243, humane chronische myelogene Leukämie), HL-60 (CCL 240, humane promyelotische Leukämie) werden in 5 % CO₂ bei 37 °C auf Kunststoffgewebezellkulturflaschen in RPMI 1640 oder DMEM mit 10 % fetalem Kalbsserum und versehen mit Penicillin zu 100 Einheiten pro ml, Streptomycin zu 100 µg pro ml, 2 mM Glutamin und 0,1 µM Dexamethason für H4IIE-Zellen gezüchtet.

Die Plasmide pCMVL und pCMVß-gal kodieren Luciferase- bzw. β-Galactosidasegene und ihre Expression steht unter der Kontrolle des Promoters des Cytomegalovirus (CMV). Die Plasmide pZeoSV und pZeoSVLacZ kodieren das Sh-Ble-resistente Protein für Zeocin (eingetragenes Warenzeichen), das eine Selektion sowohl in prokaryotische wie in eukaryotische Zellen erlaubt. Die eukaryotische Expression steht unter der Kontrolle des Promoters von CMV. pZeoSVLacZ kodiert außerdem das β-Galactosidasegen unter Kontrolle des Verstärkerpromoters von SV40. Plasmid-DNA wird auf QIAGEN-Säulen (eingetragenes Warenzeichen) unter Verwendung des QIAGEN-Plasmidreinigungsverfahrens (eingetragenes Warenzeichen) gereinigt.

Transfektion von Gewebekulturzellen. Herstellung der Zellen, Transfektionsprotokoll und Untersuchungen zur Transfektionseffizienz. Die einzelnen Transfektionen sind im Ergebnisteil ausführlich angegeben.
a) Anhaftende Zellen. Ungefähr 24 Stunden vor einer Transfektion werden fast confluente Zellmonoschichten trypsinisiert. Die Zellen werden erneut in frischem Medium suspendiert und entweder in 24-Lochplatten (5x10⁴ Zellen pro Probenvertiefung) oder in 6-Lochplatten (2-2,5x10⁴ Zellen pro Probenvertiefung) eingebracht. Unmittelbar vor der Transfektion werden die Zellen in frisches Medium mit oder ohne 10 % FCS gebracht. Im allgemeinen werden die Zellen bei 50 - 70 % Confluenz transfektiert. 24 Stunden nach der Transfektion werden die Zellen mit 2 ml PBS dreimal gewaschen und in 100 µl (24-Lochplatten) oder 200 µl (6-Lochplatten) Lysispuffer (77 mM K₂HPO₄, 23 mM KH₂PO₄, 0,2 % Triton X-100, 1 mM Dithiotreitol, pH 7,8) lysiert. Zellrückstände werden durch Zentrifugieren entfernt (14000 Upm 2 min lang). Üblicherweise wird die Enzymaktivität (siehe unten) direkt nach der Zellysis bestimmt. Die Lysate können jedoch bei -20 °C gelagert werden, ohne ihre Aktivität zu verlieren.
b) Suspensionszellen. 24 Stunden vor einer Transfektion werden die Zellen in frisches vollständiges Wachstumsmedium eingebracht. Unmittelbar vor der Transfektion werden die Zellen aufgenommen, erneut in frischem Medium mit oder ohne 10 % FCS bei 0,25x10⁶ Zellen pro ml suspendiert und in 24-Lochplatten mit 2 ml pro Probenvertiefung eingebracht.
   Die Zellen werden normalerweise 24 Stunden nach der Transfektion geerntet (die Erntezeit ist eine wichtige Größe und sollte optimiert werden), durch Zentrifugieren abgetrennt und in 10 ml PBS suspendiert, wiederum zentrifugiert und die Zellen in ein Eppendorfröhrchen von 1,5 ml mit 1 ml PBS übertragen. Die Zellen werden in der Eppendorfzentrifuge zentrifugiert (14000 Upm 20 s lang). Die erhaltenen Zellen werden in 100 µl Lysispuffer (oben beschrieben) lysiert. Die Probe wird dann zentrifugiert (14000 Upm 2 min lang) und der Überstand sorgfältig in eine frisches Zentrifugierröhrchen übertragen.
c) Transfektionsprotokolle
   Transfektion von Zellen unter Verwendung von Verbindung (1). Plasmid-DNA (aus einer Stamm-Lösung mit einer Konzentration von etwa 1 mg/ml) und kationisches Lipid (aus einer Stamm-Lösung mit einer Konzentration von 1 - 2 mg/ml) werden getrennt verdünnt in Eppendorfröhrchen von 1,5 ml mit 10 mM Hepes, 0,9 % NaCl-Puffer, pH 7,4 auf ein Endvolumen von 100 µl. Bei typischen Transfektionen schwankt die Menge an DNA von 1 bis 2 µg pro 100 µl. Die Menge eines kationischen Lipids schwankt von 0 bis 20 µg pro 100 µl. Zwei Lösungen werden zusammengemischt, so daß sich eine DNA-Lipid-Lösung bildet. Nach 10 min Inkubation bei Raumtemperatur wird die erhaltene DNA-Lipid-Lösung den Zellkulturen zugegeben, wie zuvor kultiviert und vorsichtig vermischt. Alternativ kann die DNA-Lipid-Lösung mit 800 µl (für 24- oder 6-Lochplatten) des geeigneten Mediums (+/- FCS) verdünnt und gemischt werden. Die verdünnte Lösung wird den Zellen überschichtet und mit dem geeigneten Medium gespült. Die Komplexe werden mit den Zellen 1 - 24 Stunden lang inkubiert. Eine typische Inkubationszeit in Abwesenheit von Serum beträgt 4 Stunden. Das Transfektionsmedium wird dann gegen ein vollständiges Wachstumsmedium ersetzt. In Gegenwart von Serum wird das Transfektionsmedium üblicherweise nicht ersetzt und die Zellen wachsen weiter in Gegenwart von Komplexen bis zum Ende der Versuche (einstufige Transfektion). Nach 24 Stunden werden die Zellen aufgearbeitet wie es oben beschrieben ist.
   Transfektion von Zellen unter Verwendung von ternären Komplexen mit (1)
   Plasmid-DNA wird mit 10 mM Hepes, 0,9 % NaCl-Puffer (pH 7,4), der das geeignete Peptid (beispielsweise gemäß Anspruch 25) enthält, auf ein Endvolumen von 100 µl verdünnt. Die Menge an Peptid schwankte von 0 bis 20 µg pro 100 µl Puffer. Eine typische Konzentration ist 2 - 5 µg Peptid pro 100 µl Puffer. Nach 10 min Inkubation wird die erhaltene Lösung mit 100 µl einer kationischen Lipidlösung gemischt und die ganze Vorgehensweise wie oben beschrieben fortgeführt.
d) Enzymassays
   Luciferase-Assay. Die Luciferaseaktivität in Zellextrakten wird unter Verwendung eines Berthold Lumat-Gerätes (LB 9501) durchgeführt. Der Luziferase-Assay wird durch Zugabe von 5 - 10 µl Zellextrakt durchgeführt. Die Probe wird in das LB 9501 eingebracht und das Gerät spritzt automatisch 100 µl Injektionspuffer (20 mM Tricin, 1,07 mM (MgCO₃)₄Mg(OH)₂, 2,67 mM MgSO₄, 0,1 mM EDTA, 33,3 mM DTT, 270 µM Coenzym A, 470 µM Luciferin, 530µM ATP, pH 7,8) in die Probe, mißt Lichtemission und zeigt sie als integrierten Wert für die ersten 10 Sekunden der Lichterzeugung.

β-Galactosidase-Assay. Die β-Galactosidaseaktivität in Zellextrakten wird auf 96-Loch-Mikrotiterplatten gemessen. 10 µl Zellextrakte werden mit 70 µl "β-Gal"-Puffer (33 mM NaH₂PO₄, 66 mM Na₂HPO₄, 2 mM MgSO₄, 40 mM β-Mercaptoethanol) und 25 µl ONPG-Lösung (o-Nitrophenyl-β-D-galactopyranosid 4 mg/ml in "β-Gal"-Puffer) verdünnt. Die Mischung wird 30 Minuten bis 1 Stunde lang auf 37 °C gehalten und die Reaktion durch Zusatz von 150 µl 1M Natriumbicarbonat gestoppt. Die Absorption wird bei 405 nm gemessen.

Anfärben von Zellen mit 5-Brom-4-chlor-3-indolyl-β-D-galactopyranosid (X-Gal). Die gespülten Zellen werden mit 1 %iger Glutaraldehydlösung in 0,1 M Natriumphosphat, 1 mM MgCl₂-Puffer (pH 7,0) 15 min lang bei Raumtemperatur fixiert. Die Fixierlösung wird entfernt, die Zellen dreimal mit PBS gewaschen und Anfärbelösung (0,2 % X-Gal, 10 mM Natriumphosphat, 150 mM NaCl, 1 mM MgCl₂, 3,3 mM K₄Fe(CN)₆ und 3,3 mM K₃Fe(CN)₆, pH 7,0) auf die Zellen überschichtet und 1 bis 8 Stunden bei 37 °C inkubiert. Die angefärbten Zellen auf Kulturplatten oder Objektträgern werden unter Verwendung von Inversions- bzw. Phasenkontrastmikroskopen sichtbar gemacht.

Proteinbestimmung. Der Proteingehalt im Überstand wird unter Verwendung der Technik von Lowry (Bio-Rad-Proteinbestimmungskit) untersucht.

Die Bestimmung der Zytotoxizität wird unter Verwendung des MTT-Assays (3-(4,5-Dimethylthiazol-2-yl)-2,5-diphenyltetrazoliumbromid) durchgeführt.

### Ergebnisse

### Anhaftende Zellen.

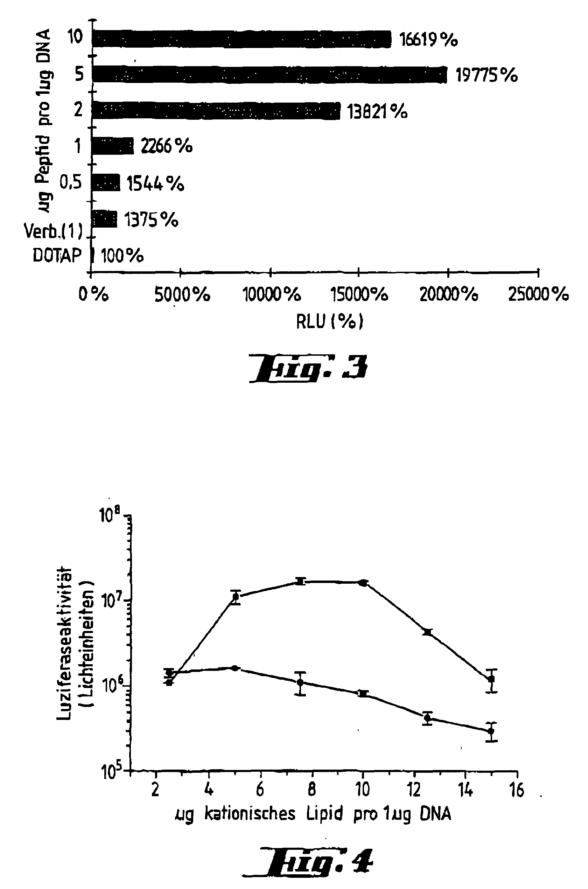

a) Verbindung (1). Die Transfektionseffizienz von Verbindung (1) auf HeLa-Zellen in Gegenwart oder Abwesenheit von Serum wird mit DOTAP verglichen. 5x10⁴ Zellen pro Probenvertiefung (24-Lochplatte) in 1 ml vollständigem RPMI 1640 werden einen Tag vor der Transfektion aufgebracht. Unmittelbar vor der Transfektion wird das Medium entfernt und die Zellen mit 1 ml frischem Medium mit oder ohne Serum versorgt. Zur Bestimmung des optimalen Verhältnisses von kationischen Lipiden zu DNA werden verschiedene Mengen beider Lipide im Transfektionspuffer (Endvolumen 100 µl) mit 100 µl DNA-Lösung gemischt, die 1 µg pCMVL enthält. Nach 10 min Inkubation bei Raumtemperatur werden 100 µl dieser Mischung, die 0,5 µg des Plasmids und die Hälfte der angegebenen Menge des Lipids enthält, zu jeder Probe zugegeben. In Gegenwart von Serum werden die Platten 24 Stunden inkubiert, ohne das Medium zu wechseln. In Abwesenheit von Serum wird das Transfektionsmedium nach 4 Stunden entfernt und die Zellen werden mit 1 ml frischem mit Serum versetztem Medium versehen.
   Die Zellen werden 24 Stunden nach der Transfektion für den Luciferase-Assay geerntet wie es oben beschrieben ist. Die Ergebnisse sind in Fig. 2 als Gesamtmenge eines kationischen Lipids pro 1 µg pCMVL angegeben. In dem Fall, wo Zellen in Gegenwart von Serum transfektiert werden, wird die optimale Expression der Luciferaseaktivität unter Verwendung eines Verhältnisses von DOTAP/DNA von 10 : 1 (Gew./Gew.) bzw. einem Verhältnis von Verbindung (1)/DNA von 5 : 1 (Gew./Gew.) erhalten. In Abwesenheit von Serum wird die optimale Expression der Luciferaseaktivität bei beiden Lipiden unter Verwendung eines Verhältnisses von Lipid/DNA von 5 : 1 (Gew./Gew.) erhalten. Die Transfektion unter Vermittlung von Verbindung (1) ist im Vergleich zur DOTAP-Transfektion in Gegenwart von Serum 12mal bzw. in Abwesenheit von Serum 6mal höher.
b) Ternärer Komplex mit Verbindung (1). 5x10⁴ Zellen pro Probenvertiefung in 1 ml vollständigem RPMI 1640 werden einen Tag vor der Transfektion aufgebracht. Unmittelbar vor der Transfektion wird das Medium entfernt und die Zellen mit 1 ml frischem Medium mit oder ohne Serum versehen. Zur Bestimmung des Einflusses von HBV-Peptid auf die Transfektion von HeLa-Zellen werden verschiedene Mengen des Peptids mit 1 µg pCMVL gemischt, so daß sich ein Endvolumen von 100 µl ergibt und 10 min inkubiert. Dann werden 100 µl einer Lösung, die 7,5 µl Verbindung (1) enthält, durch Mischen zugegeben. Nach weiteren 10 min Inkubation werden 100 µl der Transfektionslösung (enthält 0,5 µg DNA und 3,75 µg Verbindung (1)) auf die Zellen aufgebracht. Nach 4 Stunden wird das Transfektionsmedium gegen 1 ml frisches vollständiges Wachstumsmedium ersetzt.
   24 Stunden nach der Transfektion werden die Zellen lysiert und die Zellextrakte auf die Genexpression analysiert wie es oben beschrieben ist. Die Transfektionsgrade (mit Transfektion in Gegenwart von Serum) sind in Fig. 3 als Prozentsatz des mit DOTAP erhaltenen Wertes angegeben. Die Ergebnisse zeigen, daß die optimale Menge an Peptid in diesem Experiment 5 µg beträgt und die Expression der Luciferaseaktivität 14mal höher ist als die bei Transfektionen mit Verbindung (1) bzw. 20mal höher als mit DOTAP. Im Falle der Transfektion unter serumfreien Bedingungen ist die optimale Menge des Peptids gleich und die Expression der Luciferaseaktivität ungefähr 10mal höher als bei Transfektionen mit Verbindung (1) bzw. 150mal höher als mit DOTAP (Daten nicht angegeben).
c) Zeitabhängigkeit. 5x10⁴ HeLa-Zellen pro Probenvertiefung (24-Lochplatte) in 1 ml vollständigem RPMI 1640 werden einen Tag vor der Transfektion aufgebracht. Unmittelbar vor der Transfektion wird das Medium entfernt und die Zellen mit 1 ml frischem Medium mit Serum versehen. 50 µl der Transfektionslösung, die 0,25 µg pCMVLuc, 0,5 µg HBV-Peptid und 1,87 µg Verbindung (1) enthält, werden jeder Probenvertiefung unter Mischen zugegeben. Nach 2 Stunden wird das Transfektionsmedium durch 1 ml frisches vollständiges Wachstumsmedium ersetzt. Die Zellen werden zur angegebenen Zeit für das Luciferase-Assay geerntet wie es oben beschrieben ist. Die Luciferaseaktivität nimmt rasch zu, wobei sie ein Maximum nach 12 - 16 Stunden erreicht. Schon nach 2 Stunden werden 4,5x10⁴ Lichteinheiten erhalten. Dieselbe Luciferasekativität wird nach 3 Stunden mit Transfektion unter Vermittlung von Verbindung (1) und erst nach 5 - 6 Stunden bei Transfektion unter Vermittlung von DOTAP erhalten.

### Suspensionszellen.

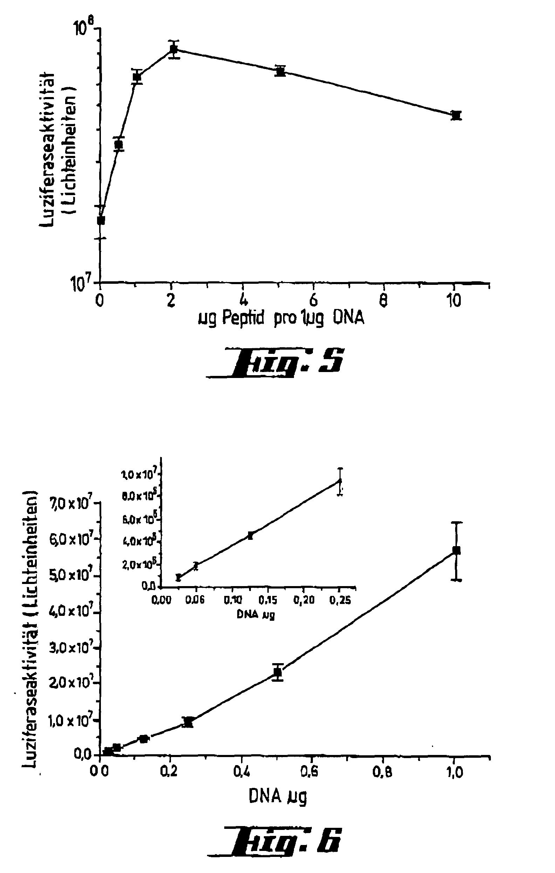

α) Verbindung (1). Die Transfektionseffizienz von Verbindung (1) auf K562-Zellen in Gegenwart von Serum wird mit DOTAP verglichen. Zur Bestimmung des optimalen Verhältnisses zwischen kationischen Lipiden und DNA werden verschiedene Mengen der beiden Lipide einer bestimmten Menge von pCMVL (1 µg pro Probenvertiefung oder pro 0,5x10⁶ Zellen in 2 ml des vollständigen RPMI 1640, 24-Lochplatten) zugegeben. Jeder Probenvertiefung werden 200 µl der Transfektionsmischung zugegeben und die Platte 24 h bei 37 °C inkubiert (einstufige Transfektion). Die Zellen werden lysiert, wie es oben beschrieben ist. Die Ergebnisse sind in Fig. 4 als Luciferasegesamtaktivität pro Probenvertiefung gegen die Menge des kationischen Lipids pro 1 µg DNa aufgetragen. Die optimale Expression der Luciferaseaktivität wird unter Verwendung eines Verhältnisses von DOTAP/DNA von 5 : 1 (Gew./Gew.) und von Verbindung (1)/DNA von 7,5 : 1 (Gew./Gew.) erhalten. Das erhaltene Verhältnis entspricht einem Ladungsverhältnis von Lipid zu DNA von ungefähr 2 : 1 für DOTAP bzw. 5 : 1 für Verbindung (1) (unter der Annahme, daß 1 µg DNA 3,1 nm anionische Phosphatladungen enthält). Die Transfektion unter Vermittlung von Verbindung (1) ist 10mal höher als mit DOTAP.
β) Ternärer Komplex mit Verbindung (1).
   Zur Bestimmung des Einflusses der Peptidkonzentration auf die Transfektion von K562-Zellen, werden verschiedene Mengen von HBV-Peptid mit 1 µg pCMVL gemischt, so daß sich ein Endvolumen von 100 µl ergibt und dann 10 min lang inkubiert. Dann werden 100 µl einer Lösung, die 10 µg Verbindung (1) enthält, unter Mischen zugegeben. Nach weiteren 10 min Inkubation wird die Transfektionslösung (200 µl) auf die Zellen aufgebracht. Alle anderen Verfahrensbedingungen sind dieselben wie im vorigen Experiment beschrieben. Die Ergebnisse sind in Fig. 5 als Luciferasegesamtaktivität pro Probenvertiefung gegen die Menge an zugesetztem Peptid (pro 1 µg DNA) aufgetragen. Die Ergebnisse zeigen, daß die optimale Menge des Peptids 2 µg beträgt und daß die Expression der Luciferase 3- bis 4mal höher ist als die bei Transfektion mit Verbindung (1) bzw. 30- bis 40mal höher als bei Transfektionen mit DOTAP.
γ) Dosisabhängigkeit.
   Eine "Stamm"-Lösung wird aus 1 µg pCMVL, 2 µg HBV-Peptid und 10 µg Verbindung (1) in einem Endvolumen von 200 µl hergestellt. Die DNA-Dosisabhängigkeit wird bei der einstufigen Transfektion durch Zugeben von 5 bis 200 µl dieser Lösung zu den Zellen geprüft. Ergebnisse dieses Versuchs sind in Fig. 6 angegeben. Die Expression der Luciferaseaktivität hängt von der Menge der zugesetzten DNA ab und ist im Bereich von 0 bis 0,25 µg DNA linear. Generell können unter diesen Bedingungen 0,5 ng Reporter-DNA (ohne Träger-DNA) nachgewiesen werden. Die erhaltenen Ergebnisse geben auch an, daß die ternären Komplexe, wenn sie einmal gebildet sind, sehr stabil sind und bei Verdünnung nicht dissoziieren.

### Intrazelluläre Einführung von Oligonukleotiden

### Beispiel 5

Fluoresceinmarkiertes Phosphothioatoligonukleotid (5'-ACT TGG ATA CGC ACG-Fluorescein-3') wird verwendet, um die intrazelluläre Abgabe und Verteilung von Oligonukleotid in Gegenwart und Abwesenheit von Verbindung (1) zu bestimmen.

HeLa-Zellen (einen Tag vor dem Experiment auf Platten aufgebracht, 2x10⁵ Zellen pro Probenvertiefung, 6-Lochplatte) werden auf Glassobjektträgern in 2 ml RPMI 1640 versehen mit 10 % FCS kultiviert. Vor dem Versuch wird das Medium durch RPMI 1640 ohne Serum ersetzt.

Die Bildung von Komplexen aus Oligonukleotid und Verbindung (1) wird wie folgt vorgenommen. 10 µg Phosphothioatoligonukleotid wird in 90 µl Transfektionspuffer aufgelöst. 16 µg und 50 µg Verbindung (1) werden mit Transfektionspuffer auf ein Endvolumen von 100 µl verdünnt. Die zwei Lösungen werden miteinander gemischt und 10 min inkubiert. Die Oligonukleotidlösung ohne Verbindung (1) wird mit 100 µl Transfektionspuffer verdünnt. Die erhaltenen Lösungen (200 µl pro Probenvertiefung) werden den Zellkulturen zugegeben (Endkonzentration des Oligonukleotids ungefähr 1 µM, Verbindung (1) 8 µM und 25 µM). Die Komplexe werden mit den Zellen 4 Stunden lang inkubiert. Dann wird das Medium durch ein vollständiges Wachstumsmedium ersetzt.

Nach 4 und 24 Stunden werden die Zellen dreimal mit PBS gewaschen und mit 1 %iger Glutaraldehydlösung 15 min lang fixiert. Nach der Fixierung werden die Zellen dreimal mit PBS gewaschen und mit Glycerinfixierlösung (10 mM Phosphat, 150 mM NaCl, 70 % Glycerin, pH 7,5) behandelt. Die Anordnung des fluoresceinmarkierten Phosphothioatoligonukleotids wird durch Fluoreszenzmikroskopie unter Verwendung eines Fluoreszenzmikroskops von Zeiss bestimmt.

Die Inkubation von Zellen mit 1 µM fluoresceinmarkiertem Oligonukleotid führte sowohl nach 4 wie nach 24 Stunden zu einer schwachen Fluoreszenz. Im Gegensatz dazu zeigten alle Zellen, die mit 1 µM fluoresceinmarkiertem Oligonukleotid in Gegenwart von Verbindung (1) inkubiert werden, bei Cytoplasmaanfärbung nach 4 Stunden eine klare Kernfluoreszenz. Nach 24 Stunden zeigten die Zellen sehr klare punktuelle Fluoreszenz im Cytoplasma. Die Fluoreszenzintensität ist in Gegenwart von 25 µM Verbindung (1) höher.

Die in Gegenwart von Serum durchgeführten Experimente zeigten keine Veränderungen der Gesamtfluoreszenz und Verteilung des fluoresceinmarkierten Oligonukleotids.

### Intrazelluläre Einführung von anionischen Polypeptiden

### Beispiel 6

Ein fluoresceinmarkiertes anionisches Polypeptid (Fluorescein-poly(Glu)₂₁) wird verwendet, um die intrazelluläre Abgabe und Verteilung von anionischem Polypeptid in Gegenwart und Abwesenheit von Verbindung (1) zu untersuchen. Die Versuchsbedingungen sind genau dieselben wie beim zuvor beschriebenen Beispiel.

Inkubation von Zellen mit 1 µM fluoresceinmarkiertem poly-(Glu)₂₁ führte weder nach 4 noch nach 24 Stunden zu einer Fluoreszenz. Im Gegensatz dazu zeigten alle mit 1 µM fluoresceinmarkiertem Oligonukleotid in Gegenwart von 25 µM Verbindung (1) inkubierten Zellen klare punktuelle Cytoplasma-Fluoreszenz schon nach 45 min. Die Anordnung der Fluoreszenzvesikel ist etwas anders als bei dem mit fluoresceinmarkiertem Oligonukleotid. Nach 24 Stunden zeigten die Zellen sehr klare punktuelle Fluoreszenz im Cytoplasma, die höchstwahrscheinlich in denselben Strukturen angeordnet sind.

### Beispiel 7

### Liposomformulierungen

Die folgenden Zusammensetzungen zeigen die Verwendung der Verbindungen dieser Erfindung in Formulierungen, die biologisch aktive Stoffe umfassen:
7.1 Eine topische Formulierung wird durch Auflösen von 0,25 mg Prednisolonacetat (21-Acetoxy-1,4-pregnadien-llβ, 17α-diol-3,20-dion) und 50 mg L-Lysin-bis(O,O'-oleoyl-β-hydroxyethyl)amiddihydrochlorid in 2 ml Dichlormethan-/Ethanollösung (1 : 1) hergestellt. Das Lösemittel wird im Stickstoffstrom verdampft. Die Filmmischung wird über Nacht im Vakuum gehalten, um das restliche Lösemittel vollständig zu verdampfen. Zur Liposomformulierung wird der trockene Film dann in 2 ml 0,9 %iger NaCl-Lösung suspendiert und die erhaltene Lösung schallbehandelt bis sie sichtbar klar ist.
7.2 (+)-α-Tocopherol (5,7,8-Trimethyltocol, Vitamin E) und 100 mg L-Lysin-bis(O,O'-oleoyl-β-hydroxyethyl)amiddihydrochlorid werden in 5 ml Dichlormethan-/Ethanollösung (1 : 1) aufgelöst. Das Lösemittel wird im Stickstoffstrom verdampft und der Rest unter Vakuum verdampft. Der trockene Film wird dann in 4 ml 10 mM Hepes, 0,9 %iger NaCl bei pH 7,4 suspendiert und schallbehandelt bis sie sichtbar klar ist.
7.3 20 mg Retinol (3,7-Dimethyl-9-(2,6,6,-trimethyl-1-cyclohexenyl)-2,4,6,8-nonatetraen-1-ol) und 80 mg L-Lysinbis(O,O'-myristoyl-β-hydroxyethyl)amiddihydrochlorid werden in 5 ml Dichlormethan-/Ethanollösung (1 : 1) aufgelöst. Das Lösemittel wird verdampft und der trockene Film über Nacht im Vakuum gehalten. Der Film wird dann in 5 ml 10 mM Tris, 0,9 %iger NaCl bei pH 7,4 suspendiert.
7.4 3 mg N-Palmitoyl-S(2,3-bis(palmitoyl-oxy)-(2RS)-propyl-R-cystein und 25 mg L-Lysin-bis(O,O'-palmitoyl-β-hydroxyethyl)amiddihydrochlorid werden in 5 ml Dichlormethan-/Ethanollösung (1 : 1) aufgelöst und unter Vakuum zur Trockene verdampft. Der erhaltene Film wird in 1 ml destilliertem Wasser suspendiert und mit Ultraschall behandelt bis die Suspension klar ist.

Neben den bisher beschriebenen Teilen umfaßt die Erfindung noch die Verwendung der erfindungsgemäßen Lipidverbindungen zur Einführung lipophiler Anteile in Peptide sowie die auf diese Weise hergestellten Lipopeptide. Nähere Einzelheiten ergeben sich aus dem folgenden:

### Peptidsynthese

### Allgemeine Vorgehensweise:

die Synthese von Peptiden mit lipophilen Modifikationen an ihren C- oder N-endständigen Gruppen oder beiden oder in der Kette wird durch schrittweise Syntheseverfahren ausgehend von Fmoc in der festen Phase unter Verwendung des automatischen Peptidsynthesegerätes ABI 350 oder des multiplen Peptidsynthesegeräts durchgeführt.

Die Peptide werden üblicherweise auf einem Rinkamid MBHA-Harz (4-(2',4'-Dimethoxyphenyl-Fmoc-aminomethyl)phenoxyacetamidonorleucylmethylbenzhydrylaminharz, Novabiochem) oder einem Tritylharz (2-Chlortritylchloridharz, Novabiochem) angeordnet.

Die Kupplungen der Fmoc-Aminosäuren (10molarer Überschuß) werden mit N,N'-Diisopropylcarbodiimid (DIC) und 1-Hydroxybenzotriazol (HOBt) in DMF üblicherweise in einer Stunde durchgeführt. Die Kupplungen der lipophilen Aminosäure-bishydroxyethylamide werden unter Verwendung eines dreifachen molaren Überschusses, mit demselben DIC/HOBt-Aktivierungsverfahren und einer Kupplungszeit von ungefähr 3 Stunden durchgeführt. Die Beendigung der Kupplung wird mit einem Ninhydrintest überprüft. Die Fmoc-Gruppe wird mit Piperidin:DMF = 1 : 2 in 15 min entfernt.

Abspaltung und Entfernung der Schutzgruppen wird in einer Mischung aus n-Kresol:Dimethylsulfid:Ethandithiol:TFA = 3:3:3:91 in zwei Stunden bei Raumtemperatur durchgeführt. Nach Abfiltrieren werden die Peptide aus der Mischung ausgefällt und mehrfach mit Diethylether gewaschen. Die rohen Peptide werden auf einer semipräparativen Säule mit Nucleosil C2 (8x250 mm) und mit einem aufsteigenden Gradienten von Acetonitril in Wasser, jeweils mit 0,1 % TFA, gereinigt. Die gereinigten Peptide werden aus der Mischung von tert-Butylalkohol:Wasser = 4:1 lyophilisiert und bei -4 °C aufbewahrt. Ihre Homogenität wird durch Aminosäureanalyse, analytische HPLC und Elektrosprayionisations-Massenspektrometrie bestätigt.

Gemäß diesem Verfahren können die folgenden Lipopeptide hergestellt werden:
GLFGAIAAGFIENGWEGLIDG-D(N(EtOPalm)₂)-NH₂
E(N(EtOPalm)₂)-MQRGNFRNQRKMVKGGRAPRKKG
E(N(EtOMyr)₂)-MQRGNFRNQRKMVKGGRAPRKKG
GRGDSPGSG-D(N(EtOPalm)₂)-NH₂
Acetyl-GRGDSPGSG-D(N(EtOPalm)₂)-NH₂
YNRNAVPNLRGDLQVLAQKVARTL-E(N(EtOPalm)₂-NH₂
E(N(EtOPalm)₂-YNRNAVPNLRGDLQVLAQKVARTL-E(N(EtOPalm)₂-NH₂
YPS-E(N(EtOPalm)₂-PDNPGEDAPAEDMARYYSALRHYINLITRQRY-NH₂

Ferner umfaßt die Erfindung noch die Verwendung der erfindungsgemäßen Lipidverbindungen zur Einführung glycolipophiler Anteile in Peptide sowie die auf diese Weise hergestellten Glycolipopeptide. Nähere Einzelheiten ergeben sich aus dem folgenden:

### Synthese von Glycolipopeptiden:

### Lys(NH-Asn(Lactosyl))₄-Lys₂-Lys-Acx-bAla-Acx-E(N(EtOPalm)₂-NH₂

1,8 g (5 mmol) D(+)-Lactosemonohydrat wird mit 40 ml gesättigter NH₄HCO₃ 6 Tage lang bei 30 °C umgesetzt. Zur Entfernung des überschüssigen NH₄HCO₃ wird die Reaktionsmischung mit Wasser (20 ml) verdünnt und im Vakuum bis auf die Hälfte ihres ursprünglichen Volumens aufkonzentriert. Dieses Vorgehen wird 6 mal wiederholt. Schließlich wird das Wasser durch Lyophilisierung entfernt. Der erhaltene rohe Aminozucker wird ohne weitere Reinigung zur Synthese von Fmoc-Asn(lactose)-OtBu-Derivaten verwendet. 480 mg (1,17 mmol) Fmoc-Asp-OtBu und 228 mg (1,52 mmol) HOBt werden in DMF gelöst (5 ml) und nach Abkühlen auf 4 °C werden 205 mg (1,63 mmol) DIC zugegeben. Nach 15 min Rühren bei 4 °C und weitere 20 min bei 25 °C werden dem aktiven Ester 5,85 mmol rohe 1-Aminolactose in 6 ml DMF:H₂O = 2:1 zugegeben. Nach 6 Stunden Rühren wird das Lösemittel im Vakuum verdampft und Diethylether zugegeben. Das ausgefällte Produkt wird filtriert, mit kaltem Ether und kaltem Wasser gewaschen. Die tBu-Schutzgruppe wird durch 70 % TFA in Wasser abgespalten (20 min bei Raumtemperatur). Nach Verdampfen der TFA-/Wassermischung im Vakuum wird das Produkt in tert-Butylalkohol:Wasser = 4:1 gelöst und lyophilisiert. Das rohe Fmoc-Asn(lactose)-OH wird durch Umkehrphasenchromatographie gereinigt (Säule Lichroprep C18 25x310 mm, isokratische Elution bei 30 % Acetonitril/Wasser/0,1 % TFA), so daß 280 mg HPLC-reines Fmoc-Asn(lactose)-OH erhalten werden (Ausbeute 35 %, bezogen auf das Ausgangsprodukt Fmoc-Asp-OtBu. Analyse: +FAB-MS (MH+) = 679 (berechnet = 679), Rt = 6,59 min auf Nucleosil C18 4x150 mm, Gradient 30-100 % Acetonitril/0,1 % TFA in Wasser/0,1 % TFA in 30 min).

32,5 mg (0,048 mmol) Fmoc-Asn(lactose)-OH und 7,25 mg (0,05 mmol) HOBt werden in 200 µl DMF gelöst und 6,31 mg (0,05 mmol) DIC zugegeben. Nach 30 min Rühren wird der gebildete aktive Ester ober Nacht mit 30 mg (0,016 mmol) Boc-(Lys(NH₂))₄-Lys₂-Lys-Acx-bAla-Acx-E(N(EtOPalm)₂-peptidylharz gekuppelt. Das Harz wird sorgfältig gewaschen und die Fmoc-Gruppe durch Behandlung mit 20 %igem Piperidin entfernt. Das Glycopeptid wird abgespalten, von Schutzgruppen befreit und unter identischen Bedingungen wie oben beschrieben aufgearbeitet. Schließlich wird es durch semipräparative HPLC gereinigt, so daß 10,6 mg N-Lactosyliertes Lipopeptid erhalten werden.

Figur 1 der beigefügten Schaubilder zeigt die Fluoreszenzintensität in Abhängigkeit von der Menge an zugesetzter Verbindung (1)

Figur 2 zeigt den Einfluß des Verhältnisses von DOTAP/DNA und Verbindung 1/DNA auf die Transfektion von HeLa-Zellen. Die Transfektion von Verbindung (1) ist in Gegenwart (■) und in Abwesenheit (□) von Serum dargestellt. Die DOTAP-Transfektion ist in Gegenwart (•) und in Abwesenheit (o) von Serum dargestellt. Die Transfektionswerte sind als Gesamtlichteinheiten pro 50000 Zellen angegeben. Jeder Punkt soll den Mittelwert ± SD von drei Transfektionen darstellen.

Figur 3 zeigt den durch Peptid verstärkten Gentransfer auf HeLa-Zellen. Transfektionswerte sind als Prozentsatz des bei DOTAP-Transfektion erhaltenen Wertes angegeben und zeigen den Mittelwert von drei Transfektionen.

Figur 4 zeigt den Einfluß des Verhältnisses von DOTAP (•)/DNA und Verbindung 1(■)/DNA auf die Transfektion von K562-Zellen. Die Transfektionswerte sind als Gesamtlichteinheiten pro 500000 Zellen angegeben. Jeder Punkt bedeutet den Mittelwert + SD von drei Transfektionen.

Figur 5 zeigt den durch Peptid verstärkten Gentransfer auf K562-Zellen. Die Transfektionswerte sind als Gesamtlichteinheiten pro 500000 Zellen angegeben und zeigen den Mittelwert ± SD von drei Transfektionen.

Figur 6 zeigt die Transfektionswerte gegen die Menge an zugesetzter DNA. Die Transfektionswerte sind als Gesamtlichteinheiten pro 500000 Zellen angegeben und bedeuten den Mittelwert ± SD von drei Transfektionen.

## Patentansprüche

1. Verbindung der Formel 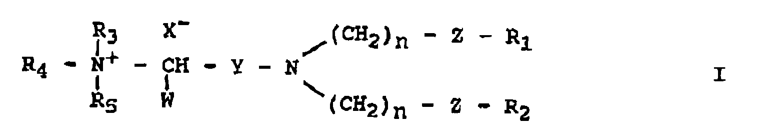 oder ein optisches Isomer davon, worin
- R₁ und R₂ gleich oder verschieden sind und eine Alkyl-, Alkenyl- oder Alkinylgruppe mit 6 bis 24 Kohlenstoffatomen sind;
- R₃ Wasserstoff, Alkyl oder Alkylamin mit 1 bis 8 Kohlenstoffatomen, oder eine Aminosäure, ein Aminosäurederivat, ein Peptid oder ein Peptidderivat ist;
- R₄ und R₅ gleich oder verschieden sind und Wasserstoff, Alkyl oder Alkylamin mit 1 bis 8 Kohlenstoffatomen sind;
- W ein seitenkettenrest von einer basischen Aminosäure und ihren Derivaten, Peptiden oder peptidderivaten ist;
- Y -CO-, -(CH₂)ₘCO-, -(CH₂-)ₘ, -(CHOHCH₂-)ₘ, worin m gleich 1 bis 20 ist, -CH₂S-CH₂-, -CH₂-SO-CH₂, -CH₂-SO₂-CH₂-, -CH₂-SO₂- oder -SO₂- ist;
- Z eine Ester-, Ether- oder Amidbindung ist;
- n gleich 1 bis 8 ist; und
- X ein Anion ist.

2. Verbindung nach Anspruch 1, **dadurch gekennzeichnet, dass** Y Carbonyl, d.h. -CO- ist.

3. Verbindung nach Anspruch 1 oder 2, **dadurch gekennzeichnet, dass** Z eine Esterbindung ist.

4. Verbindung nach einem der vorhergehenden Ansprüche, **dadurch gekennzeichnet, dass** R₁ und R₂ Alkyl- oder Alkenylgruppen mit 10 bis 20 Kohlenstoffatomen sind.

5. Verbindung nach Anspruch 4, **dadurch gekennzeichnet, dass** R₁ und R₂ Alkyl- oder Alkenylgruppen mit 12 bis 18 Kohlenstoffatomen sind.

6. Verbindung nach Anspruch 4 oder 5, **dadurch gekennzeichnet, dass** R₁ und R₂ gleich sind.

7. Verbindung nach einem der vorhergehenden Ansprüche, **dadurch gekennzeichnet, dass** R₁ und R₂ eine Alkenylgruppe sind.

8. Verbindung nach einem der vorhergehenden Ansprüche, **dadurch gekennzeichnet, dass** n gleich 2 ist.

9. Verbindung nach einem der vorhergehenden Ansprüche, **dadurch gekennzeichnet, dass** W der Seitenkettenrest von Lysin oder Ornithin ist.

10. Verbindung nach einem der vorhergehenden Ansprüche, **dadurch gekennzeichnet, dass** R₃, R₄ und R₅ Wasserstoff sind.

11. Verbindung nach eines der vorhergehenden Ansprüche, **dadurch gekennzeichnet, dass** x das Chloridion ist.

12. Verbindung nach einem der vorhergehenden Ansprüche, **dadurch gekennzeichnet, dass** die Verbindung ausgewählt ist aus
L-Lysin-bis-(O,O'-cis-9-octadecenoyl-β-hydroxyethyl)-amiddihydrochlorid oder einem optischen Isomer davon, L-Lysin-bis-(O,O'-haxadecanoyl-β-hydroxyethyl)amiddihydrochlorid oder einem optischen Isomer davon, L-Ornithin-bis-(O,O-cis-9-octadecenoyl-β-hydroxyethyl)-amiddihydrochlorid oder einem optischen Isomer davon, L-Ornithin-bis-(O,O'-hexadecanoyl-β-hydroxyethyl)amiddihydrochlorid oder einem optischen Isomer davon, L-Lysin-bis-(O,O'-tetradecanoyl-β-hydroxyethyl)amiddihydrochlorid oder einem optischen Isomer davon, L-Ornithin-bis-(O,O'-tetradecanoyl-β-hydroxyethyl)amiddihydrochlorid oder einem optischen Isomer davon.

13. Verbindung nach minen der vorhergehenden Ansprüche, **dadurch gekennzeichnet, dass** die Verbindung L-Lysin-bis-(O,O'-cis-9-octadecenoly-β-hydroxyethyl)amiddihydrochlorid oder ein optisches Isomer davon ist.

14. Polyanion-Lipid-Komplex aus einem Polyanion und mindestens einer Verbindung nach einen der Ansprüche 1 bis 13.

15. Komplex nach Anspruch 14, **dadurch gekennzeichnet, dass** er eine positive Gesamt- bzw. Nettoladung besitzt.

16. Komplex nach Anspruch 13 oder 14, **dadurch gekennzeichnet, dass** das Polyanion ein Polynukleotid ist.

17. Komplex nach Anspruch 16, **dadurch gekennzeichnet, dass** das Polyanion DNA (Desoxyribonukleinsäure) oder RNA (Ribonukleinsäure) ist.

18. Komplex nach einem der Ansprüche 14 oder 15, **dadurch gekennzeichnet, dass** das Polyanion ein Polypeptid ist.

19. Komplex nach einem der Ansprüche 14 bis 18, **dadurch gekennzeichnet, dass** das Lipid die Verbindung nach Anspruch 13 oder ein optisches Isomer davon ist.

20. Ternärer Polyanion-Polykation-Lipid-Komplex aus einem Polyanion, einem Polykation und mindestens einer Verbindung nach einem der Ansprüche 1 bis 13.

21. Komplex nach Anspruch 20, **dadurch gekennzeichnet, dass** er eine positive Gesamt- bzw. Nettoladung besitzt.

22. Komplex nach Anspruch 20 oder 21, **dadurch gekennzeichnet, dass** das Polyanion ein Polynukleotid ist.

23. Komplex nach Anspruch 22, **dadurch gekennzeichnet, dass** das Polyanion DNA (Desoxyribonukleinsäure) oder RNA (Ribonukleinsäure) ist.

24. Komplex nach einem der Ansprüche 20 bis 23, **dadurch gekennzeichnet, dass** das Polykation ein Polypeptid ist.

25. Komplex nach Anspruch 24, **dadurch gekennzeichnet, dass** das Peptid mindestens eine der folgenden Aminosäureaequenzen umfaßt:
- a) GRSPRRRTPSPRRRRSQSPRRRRSQS,
- b) RRRRSQSPRRRRSQS,
- c) PKKKRKVPGSGRSPRRRTPSPRKRRSQSPRRRRSQS
- d) PKKKRKVPGSGRRRRSQSPRRKRSQS
- e) GRAPKRRTPAPRRRRAQAPRRRRAQA,
- mine Protaminsequenz,
- eine Histonsequenz.

26. Komplex nach einem der Ansprüche 20 bis 25, **dadurch gekennzeichnet, dass** das Lipid die Verbindung nach Anspruch 13 oder ein optisches Isomer davon ist.

27. Verfahren zur Herstellung eines Komplexes nach einem der Ansprüche 14 bis 19, **dadurch gekennzeichnet, dass** mindestens eine Verbindung nach einem der Ansprüche 1 bis 13 oder eine Zusammensetzung, die mindestens eine solche Verbindung enthält, mit einem Polyanion in Kontakt gebracht wird.

28. Verfahren zur Herstellung eines Komplexes nach einem der Ansprüche 20 bis 26, **dadurch gekennzeichnet, dass** das Polyanion mit dem Polykation in Kontakt gebracht wird und der daraus resultierende Polyanion-Polykation-Komplex mit mindestens einer Verbindung nach einem der Ansprüche 1 bis 13 oder einer Zusammensetzung, die diese Verbindung enthält, in Kontakt gebracht wird.

29. Liposom hergestellt mit mindestens einer Verbindung nach einem der Ansprüche 1 bis 13.

30. Liposom-Formulierung in wässriger Lösung umfassend
- mindestens eine biologisch aktive Substanz, und
- eine Lipidkomponente, enthaltend mindestens eine Verbindung nach einem der Ansprüche 1 bis 13.

31. Liposom-Formulierung nach Anspruch 30, **dadurch gekennzeichnet, dass** die biologisch aktive Substanz in einer Menge bis zu 10 Gew.-% vorhanden ist.

32. Liposom-Formulierung nach Anspruch 30 oder 31, **dadurch gekennzeichnet, dass** die Lipidkomponente in einer Menge von 1 bis 20 Gew.-% vorhanden ist.

33. Liposom-Formulierung nach einem der Ansprüche 30 bis 32, **dadurch gekennzeichnet, dass** die Verbindung in der Lipidkomponente 1 % bis 100 % dieser Komponente umfaßt.

34. Liposom-Formulierung nach einem der Ansprüche 30 bis 33, **dadurch gekennzeichnet, dass** die biologisch aktive Substanz ein Arzneistoff ist.

## Claims

1. Compound according to the formula 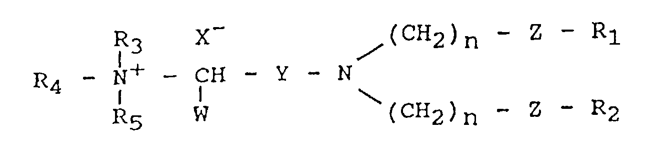 or an optical isomer thereof, wherein
- R₁ and R₂ are the same or different and are an alkyl, alkenyl, or alkynyl group of 6 to 24 carbon atoms;
- R₃ is hydrogen, alkyl or alkylamine with 1 to 8 carbon atoms, or an amino acid, an amino acid derivative, a peptide or a peptide derivative,
- R₄ and R₅ are the same or different and are hydrogen, alkyl or alkylamine with 1 to 8 carbon atoms;
- W is a side chain group of a basic amino acid and its derivatives, peptides or peptide derivatives;
- Y is -CO, -(CH₂)ₘCO-, -(CH₂)m, -(CHOHCH₂)ₘ, wherein m is from 1 to 20, -CH₂-S-CH₂-, -CH₂SO-CH₂, -CH₂-SO₂-CH₂-, -CH₂-SO₂- or -SO₂-;
- Z is an ester, ether, or amide bond;
- n is from 1 to 8; and
- X is an anion.

2. Compound according to claim 1, **characterized in that** Y is carbonyl, that is -CO-.

3. Compound according to claim 1 or 2, **characterized in that** Z is an ester bond.

4. Compound according to any of the preceding claims, **characterized in that** R₁ and R₂ are alkyl or alkenyl groups having from 10 to 20 carbon atoms.

5. Compound according to claim 4, **characterized in that** R₁ and R₂ are alkyl or alkenyl groups with 12 to 18 carbon atoms.

6. Compound according to claim 4 or 5, **characterized in that** R₁ and R₂ are the same.

7. Compound according to any of the preceding claims, **characterized in that** R₁ and R₂ are an alkenyl group.

8. Compound according to any of the preceding claims, **characterized in that** n is 2.

9. Compound according to any of the preceding claims, **characterized in that** W is the side chain group of lysine or ornithine.

10. Compound according to any one of the preceding claims, **characterized in that** R₃, R₄ and R₅ are hydrogen.

11. Compound according to any of the preceding claims, **characterized in that** X is the chloride anion.

12. Compound according to any of the preceding claims, **characterized in that** the compound is selected from L-lysine-bis-(O,O' -cis-9-octadecenoyl-β-hydroxyethyl)-amide dihydrochloride or an optical isomer thereof, L-lysine-bis-(O,O'-hexadecanoyl-β-hydroxyethyl)-amide dihydrochloride or an optical isomer thereof, L-ornithine-bis-(O,O'-cis-9-octadecenoyl-β-hydroxyethyl) amide dihydrochloride or an optical isomer thereof, L-omithine-bis-(O,O'-hexadecanoyl-β-hydroxyethyl) amide dihydrochloride or an optical isomer thereof, L-lysine-bis-(O-O'-tetradecanoyl-β-hydroxyethyl) amide dihydrochloride or an optical isomer thereof, L-ornithine-bis-(O,O'-tetradecanoyl-β-hydroxyethyl)-amide dihydrochloride or an optical isomer thereof.

13. Compound according to any of the preceding claims, **characterized in that** the compound is L-lysine-bis-(O,O'-cis-9-octadecenoyl-β-hydroxyethyl) amide dihydrochloride or an optical isomer thereof.

14. Polyanion-lipid complex composed of a polyanion and at least one compound according to any of the claims 1 to 13.

15. Complex according to claim 14, **characterized in that** it has a positive total or net charge.

16. Complex according to claim 13 or 14, **characterized in that** the polyanion is a polynucleotide.

17. Complex according to claim 16, **characterized in that** the polyanion is DNA (deoxyribonucleic acid) or RNA (ribonucleic acid).

18. Complex according to claim 14 or 15, **characterized in that** the polyanion is a polypeptide.

19. Complex according to any of claims 14 to 18, **characterized in that** the lipid is the compound according to claim 13 or an optical isomer thereof.

20. Ternary polyanion-polycation-lipid complex composed of a polyanion, a polycation, and at least one compound according to any of the claims 1 to 13.

21. Complex according to claim 20, **characterized in that** it has a positive total or net charge.

22. Complex according to claim 20 or 21, **characterized in that** the polyanion is a polynucleotide.

23. Complex according to claim 22, **characterized in that** the polyanion is DNA (deoxyribonucleic acid) or RNA (ribonucleic acid).

24. Complex according to any of the claims 20 to 23, **characterized in that** the polycation is a polypeptide.

25. Complex according to claim 24, **characterized in that** the peptide comprises at least one of the following amino acid sequences:
- a) GRSPRRRTPSPRRRRSQSPRRRRSQS,
- b) RRRRSQSPRRRRSQS
- c) PKKKRKVPGSGRSPRRRTPSPRRRRSQSPRRRRSQS,
- d) PKKKRKVPGSGRRRRSQSPRRRRSQS
- e) GRAPRRRTPAPRRRRAQAPRRRRAQA
- a protamine sequence,
- a histone sequence.

26. Complex according to any of the claims 20 to 25, **characterized in that** the lipid is the compound according to claim 13 or an optical isomer thereof.

27. Method for the preparation of a complex according to any of claims 14 to 19, **characterized in that** at least one compound according to any of claims 1 to 13, or a composition including at least one such compound is contacted with a polyanion.

28. Method for the preparation of a complex according to any of claims 20 to 26, **characterized in that** the polyanion is contacted with the polycation and the resulting polyanion-polycation complex is contacted with at least one compound according to any of claims 1 to 13, or a composition including this compound.

29. Liposome prepared with at least one compound according to any of claims 1 to 13.

30. Liposome formulation including
- at least one biologically active substance, and
- a lipid component, including at least one compound according to any of claims 1 to 13.

31. Liposome formulation according to claim 30, **characterized in that** the biologically active substance is present in an amount up to 10 % by weight.

32. Liposome formulation according to claim 30 or 31, **characterized in that** the lipid component is present in an amount from 1 to 20 % by weight.

33. Liposome formulation according to any of claims 30 to 32, **characterized in that** the compound in the lipid component constitutes from 1 % to 100 % of this component.

34. Liposome formulation according to any of claims 30 to 33, **characterized in that** the biologically active substance is a drug.

## Revendications

1. Composition de la formule 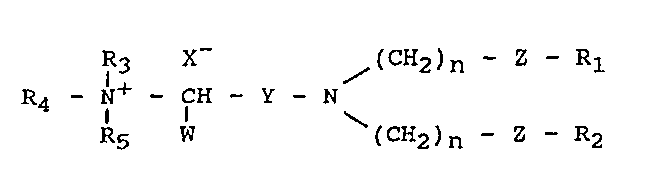 où un isomère optique de cette dernière dans lequel
- R₁ et R₂ sont identiques ou différents et un groupe d'alkyle, d'alkényle ou d'alkinyle avec 6 à 24 atomes de carbone ;
- R3 est l'hydrogène, l'alkyle ou l'alkylamine avec 1 à 8 atomes de carbone, ou un acide aminé ou un dérivé d'acide aminé, un peptide ou un dérivé de peptide ;
- R4 et R5 sont identiques ou différents et sont de l'hydrogène, de l'alkyle ou de l'alkylamine avec 1 à 8 atomes de carbone ;
- W est un reste de chaîne latérale d'un acide aminé basique et de leurs dérivés, peptides ou dérivés de peptides ;
- Y -CO-, -(CH₂)ₘCO-, -(CH₂-)ₘ, -(CHOHCH₂-)ₘ, où m est égal de 1 à 20, -CH₂S-CH₂-, -CH₂-SO-CH₂, -CH₂-SO₂-CH₂-, est -CH₂-SO₂ ou -SO₂ -;
- Z est une liaison d'ester, d'éther ou d'amide ; et
- n a une valeur de 1 à 8 ; et
- X est un anion.

2. Composé selon la revendication 1, **caractérisé en ce que** Y est du carbonyle, c'est-à-dire -CO-.

3. Composé selon la revendication 1 ou 2, **caractérisé en ce que** Z est une liaison d'ester.

4. Composé selon l'une des revendications précédentes, **caractérisé en ce que** R₁ et R₂ sont des groupes d'alkyle ou d'alkényle avec 10 à 20 atomes de carbone.

5. Composé selon la revendication 4, **caractérisé en ce que** R₁ et R₂ sont des groupes d'alkyle ou d'alkényle avec 12 à 18 atomes de carbone.

6. Composé selon la revendication 4 ou 5, **caractérisé en ce que** R₁ et R₂ sont identiques.

7. Composé selon l'une des revendications précédentes **caractérisé en ce que** R1 et R2 sont un groupe d'alkényle.

8. Composé selon l'une des revendications précédentes, **caractérisé en ce que** n est égale à 2.

9. Composé selon l'une des revendications précédentes, **caractérisé en ce que** W est le reste de chaîne latérale de lysine ou d'ornithine.

10. Composé selon l'une des revendications précédentes, **caractérisé en ce que** R3, R4 et R5 sont de l'hydrogène.

11. Composé selon l'une des revendications précédentes, **caractérisé en ce que** X est le chlorure ionique.

12. Composé selon l'une des revendications précédentes, **caractérisé en ce que** le composé est sélectionné parmi L-Lysine-bis-(O,O'-cis-9-octadecenoyl-β-hydoxyéthyle)-amiddihydrochlorure ou un isomère optique de ces derniers, L-Lysine-bis-(O,O'-hexadécanoyl-β-hydroxyéthyle)amid-dihydrochlorure ou un isomère optique de ces derniers, L-Ornithine-bis-(O,O'-cis-9-octadecenoyl-β-hydoxyéthyle)-amiddihydrochlorure ou un isomère optique de ces derniers, L-Ornithine-bis-(O,O'-hexadécanoyl-β-hydroxyéthyle)amid-dihydrochlorure ou un isomère optique de ces derniers, L-Lysine-bis-(O,O'-tétradécanoyl-β-hydroxyéthyle)amid-dihydrochlorure ou un isomère optique de ces derniers, L-Ornithine-bis-(O,O'-tétradécanoyl-β-hydroxyéthyle)amid-dihydrochlorure ou un isomère optique de ces derniers.

13. Composé selon l'une des revendications précédentes, **caractérisé en ce que** le composé est L-Lysine-bis-(O,O'-cis-9-octadecenoyl-β-hydoxyéthyle)-amiddihydrochlorure ou un isomère optique de ces derniers.

14. Complexe lipidique polyanionique à partir d'un polyanion et d'au moins un composé selon l'une des revendications 1 à 13.

15. Complexe selon la revendication 14, **caractérisé en ce qu'**il possède une charge positive nette respectivement totale.

16. Complexe selon la revendication 13 ou 14, **caractérisé en ce que** le polyanion est un polynucléotide.

17. Complexe selon la revendication 16, **caractérisé en ce que** le polyanion est du DNA (acide désoxyribonucléique) ou du RNA (acide ribonucléique).

18. Complexe selon l'une des revendications 14 ou 15, **caractérisé en ce que** le polyanion est un polypeptide.

19. complexe selon l'une des revendications 14 à 18, **caractérisé en ce que** le lipide est le composé selon la revendication 13 ou un isomère optique.

20. Complexe lipidique ternaire de polyanion-polycation se composant d'un polyanion, d'un polycation et au moins d'un composé selon l'une des revendications 1 à 13.

21. Complexe selon la revendication 20, **caractérisé en ce qu'**il possède une charge positive nette respectivement totale.

22. Complexe selon la revendication 20 ou 21, **caractérisé en ce que** le polyanion est un polynucléotide.

23. Complexe selon la revendication 22, **caractérisé en ce que** le polyanion est du DNA (acide désoxyribonucléique) ou du RNA (acide ribonucléique).

24. Complexe selon l'une des revendications 20 à 23 **caractérisé en ce que** le polycation est polypeptide.

25. Complexe selon la revendication 24, **caractérisé en ce que** le peptide comprend au moins l'une des séquences suivantes d'acides aminés :
- a) GRSPRRRTPSPRRRRSQSPRRRRSQS,
- b) RRRRSQSPRRRRSQS
- c) PKKKRKVPGSGRSPRRRTPSPRRRRSQSPRRRRSQS
- d) PKKKRKVPGSGRRRRSQSPRRRRSQS
- e) GRAPRRRTPAPRRRRAQAPRRRRAQA
- une séquence de protamine
- une séquence histonique.

26. Complexe selon l'une des revendications 20 à 25, **caractérisé en ce que** le lipide est le composé selon la revendication 13 ou un isomère optique de ce dernier.

27. Procédé de fabrication d'un complexe selon l'une des revendications 14 à 19, **caractérisé en ce qu'**au moins un composé selon l'une des revendications 1 à 13, ou une composition qui contient au moins un tel composé est amené en contact avec un polyanion.

28. Procédé de fabrication d'un complexe selon l'une des revendications 20 à 26, **caractérisé en ce que** le polyanion est amené en contact avec le polycation et le complexe en résultant de polyanion et de polycation est amené en contact avec au moins un composé selon l'une des revendications 1 à 13 ou une composition qui contient ce composé.

29. Liposome fabriqué avec au moins un composé selon l'une des revendications 1 à 13.

30. Formulation de liposome en solution aqueuse comprenant
- au moins une substance active biologiquement et
- un composant lipidique, contenant au moins un composé selon l'une des revendications 1 à 13.

31. Formulation de liposome selon la revendication 30, **caractérisé en ce que** la substance biologiquement active est présente dans une quantité pondérale maximale de 10 %.

32. Formulation de liposome selon la revendication 30 à 31, **caractérisé en ce que** le composant lipidique est présent dans une quantité pondérale de 1 à 20 %.

33. Formulation de liposome selon la revendication 30 à 32, **caractérisé en ce que** le composé comprend dans le composant lipidique 1 à 100 % de ce composant.

34. Formulation de liposome selon la revendication 30 ou 33, **caractérisé en ce que** la substance biologiquement active est un médicament.
